# EUROPEAN PATENT APPLICATION

(11) **EP 4 506 365 A1**
(43) Date of publication of application: **12.02.2025**
(21) Application number: 23778445.9
(22) Date of filing: 31.03.2023
(51) Int. Cl.: C07K 16/46, C07K 16/28, C12N 15/13, C12N 15/63, C12N 5/10, A61K 39/395, A61P 35/00

(54) **ANTI-TIGIT/ANTI-PVRIG BISPECIFIC ANTIBODY, AND PHARMACEUTICAL COMPOSITION AND USE THEREOF**

(30) Priority: 02.04.2022 CN 202210345707
(71) Applicant: Biotheus Inc., Tangjiawan Town, Xiangzhou District Zhuhai, Guangdong 519080 (CN)
(72) Inventor: ZHAI, Tianhang, Zhuhai, Guangdong 519080 (CN); DAI, Shuang, Zhuhai, Guangdong 519080 (CN); HUANG, Weifeng, Zhuhai, Guangdong 519080 (CN); MIAO, Xiaoniu, Zhuhai, Guangdong 519080 (CN); TSUN, Andy, Zhuhai, Guangdong 519080 (CN)
(74) Representative: Ghirardi, Valeria
(86) International application number: PCT/CN2023/085428
(87) International publication number: WO 2023/186081

(57) **Abstract**

The present invention belongs to the field of biological medicines, and relates to an anti-TIGIT-anti-PVRIG bispecific antibody, and a pharmaceutical composition and use thereof. Specifically, the present invention relates to a bispecific antibody, comprising: a first protein functional region targeting PVRIG and a second protein functional region targeting a target (e.g., TIGIT) different from PVRIG, wherein the first protein functional region is an anti-PVRIG immunoglobulin or an antigen-binding fragment thereof; a heavy chain variable region of the anti-PVRIG immunoglobulin comprises HCDR1 having an amino acid sequence as set forth in SEQ ID NO: 25, HCDR2 having an amino acid sequence as set forth in SEQ ID NO: 26, and HCDR3 having an amino acid sequence as set forth in SEQ ID NO: 27; and a light chain variable region of the anti-PVRIG immunoglobulin comprises LCDR1 having an amino acid sequence as set forth in SEQ ID NO: 22, LCDR2 having an amino acid sequence as set forth in SEQ ID NO: 23, and LCDR3 having an amino acid sequence as set forth in SEQ ID NO: 24. The bispecific antibody of the present invention has a good anti-tumor effect.

## Description

### Technical Field

The present invention belongs to the field of biomedicine and relates to an anti-TIGIT-anti-PVRIG bispecific antibody, pharmaceutical composition thereof and use thereof.

### Background Art

T cell immunoreceptor with Ig and ITIM domains (TIGIT, also known as WUCAM, Vstm3, VSIG9) is a novel immunosuppressive receptor expressed by activated CD8⁺ T and CD4⁺ T cells, natural killer (NK) cells, regulatory T cells (Tregs) and follicular helper T cells.

Poliovirus receptor related immunoglobulin domain-containing protein (PVRIG, also known as CD112R) is a member of the PVR family with an IgV structural domain in the extracellular region and an immunoreceptor tyrosine-based inhibitor motif (ITIM) in the intracellular region, and is a novel immunosuppressive receptor expressed on NK cells and T cells.

PVRIG or its ligand PVRL2 is highly expressed in lung, kidney, endometrial, breast and skin cancers and their tumor microenvironments, and PVRIG was also highly expressed on NK cells from prostate cancer patients (Whelan S, et al. Cancer Immunol Res. 2019;7(2):257-268.). Additionally, PVRIG is co-expressed with the exhaustion markers TIGIT and PD-1 on CD8+ T cells, suggesting that PVRIG expression has certain tumor specificity and may be associated with the activated/exhausted state of TILs (Whelan S, et al. Cancer Immunol Res. 2019;7(2):257-268.). WO2021180205A1 reported that anti-PVRIG antibodies can be used for the treatment of lung cancer, breast cancer, ovarian cancer, kidney cancer, gastric cancer, endometrial cancer and head and neck cancer.

TIGIT/PVRIG are involved in a complex regulatory network: CD226 (DNAM-1) is an activating receptor on T/NK cells that mediates activation signals by binding to CD155 and CD112; TIGIT and PVRIG are inhibitory receptors on T/NK cells that are upregulated upon cell activation and independently introduce inhibitory signals by binding to the ligands CD155 and CD112, respectively, while also competing with CD226 for ligand binding, thereby blocking its stimulatory signals and achieving negative regulation of immune cell function.

Researchers found that blocking the interactions of TIGIT/CD155 and PVRIG/CD112 with antibodies separately could enhance the cytotoxicity of NK cells against tumor cells, and the combination of PVRIG and TIGIT blocking monoclonal antibodies could further enhance the killing effect of NK cells on breast cancer cells (Xu F, et al. Blockade of CD112R and TIGIT signaling sensitizes human natural killer cell functions. Cancer Immunol Immunother. 2017 Oct;66(10): 1367-1375.). In vitro functional studies of T cells showed that TIGIT antibody and PVRIG antibody could individually increase T cell expansion and IFN-γ secretion, respectively, while the combination of the two could produce additive or synergistic effects, further enhancing T cell function (Whelan S, et al. PVRIG and PVRL2 Are Induced in Cancer and Inhibit CD8+ T-cell Function. Cancer Immunol Res. 2019 Feb;7(2):257-268.). In a knockout mouse model, PVRIG antibody reduced tumor growth in TIGIT-/- mice, and tumor growth control was further improved in PVRIG and TIGIT double knockout mice compared to single gene knockout mice (Kathryn Logronio, et al. COM902, a Novel Therapeutic Antibody Targeting TIGIT Augments T Cell Function and the Activity of PVRIG Pathway Blockade In Vitro and In Vivo. SITC 2019.). These experimental data have demonstrated that TIGIT/CD155 and PVRIG/CD112 are two independent, non-redundant T cell inhibitory pathways. Simultaneous targeting both TIGIT and PVRIG holds great promise for better unleashing the T cell activation signals generated by CD226 in cancer therapy.

SHR-2002, developed by Hengrui Pharmaceuticals, is the first and only TIGIT/PVRIG dual-target antibody of its kind to enter clinical development. It was approved for clinical development in early December 2021 and is currently in Phase I clinical trials for the treatment of cancer.

Simultaneous targeting of TIGIT and PVRIG has shown good results in both in vivo and in vitro experiments. Bispecific antibodies can specifically bind two antigens or antigenic epitopes simultaneously, which is characterized by specificity and bifunctionality, and have become a research hotspot in the field of antibody engineering. However, the problems encountered in the development of bispecific antibodies, such as complex preclinical evaluation models, low expression, poor stability, complicated processes, and high variability in quality control, have affected the progress of bispecific antibody development.

Therefore, there is still an urgent need to develop a bispecific antibody targeting TIGIT and PVRIG with good specificity, efficacy and easy to prepare.

### Contents of the present invention

After intensive research and creative work, the inventors obtained a novel bispecific antibody. The inventors surprisingly found that the bispecific antibody of the present invention exhibits excellent affinity and biological activity, with potential for anti-tumor effects. This has led to the following inventions:
One aspect of the present invention relates to a bispecific antibody, which comprises:
a first protein functional region targeting PVRIG, and
a second protein functional region targeting a target (e.g., TIGIT) different from PVRIG; wherein:
   the first protein functional region is an anti-PVRIG immunoglobulin or an antigen-binding fragment thereof;
   the heavy chain variable region of the anti-PVRIG immunoglobulin comprises HCDR1 with the amino acid sequence as set forth in SEQ ID NO: 25, HCDR2 with the amino acid sequence as set forth in SEQ ID NO: 26, and HCDR3 with the amino acid sequence as set forth in SEQ ID NO: 27; and
   the light chain variable region of the anti-PVRIG immunoglobulin comprises LCDR1 with the amino acid sequence as set forth in SEQ ID NO: 22, LCDR2 with the amino acid sequence as set forth in SEQ ID NO: 23, and LCDR3 with the amino acid sequence as set forth in SEQ ID NO: 24.

In some embodiments of the present invention, the bispecific antibody is anti- TIGIT -anti-PVRIG bispecific antibody, also referred to as anti-PVRIG-anti-TIGIT bispecific antibody, and is abbreviated as the bispecific antibody of the present invention.

In some embodiments of the present invention, the bispecific antibody is provided, wherein the heavy chain variable region of the anti-TIGIT immunoglobulin comprises HCDR1 with the amino acid sequence as set forth in SEQ ID NO: 13, HCDR2 with the amino acid sequence as set forth in SEQ ID NO: 14, and HCDR3 with the amino acid sequence as set forth in SEQ ID NO: 15; and the light chain variable region of the anti-TIGIT immunoglobulin comprises LCDR1 with the amino acid sequence as set forth in SEQ ID NO: 16, LCDR2 with the amino acid sequence as set forth in SEQ ID NO: 17, and LCDR3 with the amino acid sequence as set forth in SEQ ID NO: 18;
or,
the heavy chain variable region of the anti-TIGIT immunoglobulin comprises HCDR1 with the amino acid sequence as set forth in SEQ ID NO: 19, HCDR2 with the amino acid sequence as set forth in SEQ ID NO: 20, and HCDR3 with the amino acid sequence as set forth in SEQ ID NO: 21; and the light chain variable region of the anti-TIGIT immunoglobulin comprises LCDR1 with the amino acid sequence as set forth in SEQ ID NO: 22, LCDR2 with the amino acid sequence as set forth in SEQ ID NO: 23, and LCDR3 with the amino acid sequence as set forth in SEQ ID NO: 24.

The variable regions of the light chain and the heavy chain determine the binding to antigen; the variable region of each chain comprises three hypervariable regions, called complementarity determining regions (CDRs), of which the CDRs of the heavy chain (H) include HCDR1, HCDR2, and HCDR3, and the CDRs of the light chain (L) include LCDR1, LCDR2, and LCDR3. In some embodiments of the present invention, HCDR1-HCDR3 and LCDR1-LCDR3 are defined or numbered according to the United States Patent Publication US20210380669A1 or the literature Lu et al (Deamidation and isomerization liability analysis of 131 clinical-stage antibodies, MABS, 2019, VOL. 11, NO. 1, 45-57, DOI: 10.1080/19420862.2018.1548233) .

In some embodiments of the present invention, the bispecific antibody is provided, wherein, the antigen-binding fragment thereof is independently a single-chain antibody or an IgG half molecule (IgG-HM).

In some embodiments of the present invention, the bispecific antibody is provided, wherein,
the first protein functional region is an anti-PVRIG immunoglobulin, and the second protein functional region is an anti-TIGIT single-chain antibody; wherein:
the heavy chain variable region of the anti-PVRIG immunoglobulin comprises HCDR1 with the amino acid sequence as set forth in SEQ ID NO: 25, HCDR2 with the amino acid sequence as set forth in SEQ ID NO: 26 and HCDR3 with the amino acid sequence as set forth in SEQ ID NO: 27; and the light chain variable region of the anti-PVRIG immunoglobulin comprises LCDR1 with the amino acid sequence as set forth in SEQ ID NO: 22, LCDR2 with the amino acid sequence as set forth in SEQ ID NO: 23, and LCDR3 with the amino acid sequence as set forth in SEQ ID NO: 24; and
the heavy chain variable region of anti-TIGIT single-chain antibody comprises HCDR1 with the amino acid sequence as set forth in SEQ ID NO: 13, HCDR2 with the amino acid sequence as set forth in SEQ ID NO: 14, and HCDR3 with the amino acid sequence as set forth in SEQ ID NO: 15; and the light chain variable region of the anti-TIGIT immunoglobulin comprises LCDR1 with the amino acid sequence as set forth in SEQ ID NO: 16, LCDR2 with the amino acid sequence as set forth in SEQ ID NO: 17, and LCDR3 with the amino acid sequence as set forth in SEQ ID NO: 18; or
the heavy chain variable region of anti-TIGIT single-chain antibody comprises HCDR1 with the amino acid sequence as set forth in SEQ ID NO: 19, HCDR2 with the amino acid sequence as set forth in SEQ ID NO: 20, and HCDR3 with the amino acid sequence as set forth in SEQ ID NO: 21; and the light chain variable region of the anti-TIGIT immunoglobulin comprises LCDR1 with the amino acid sequence as set forth in SEQ ID NO: 22, LCDR2 with the amino acid sequence as set forth in SEQ ID NO: 23, and LCDR3 with the amino acid sequence as set forth in SEQ ID NO: 24.

In some embodiments of the present invention, the bispecific antibody is provided, wherein:
the first protein functional region is an anti-PVRIG single-chain antibody, and the second protein functional region is an anti-TIGIT immunoglobulin; wherein:
the heavy chain variable region of the anti-PVRIG single-chain antibody comprises HCDR1 with the amino acid sequence as set forth in SEQ ID NO: 25, HCDR2 with the amino acid sequence as set forth in SEQ ID NO: 26, and HCDR3 with the amino acid sequence as set forth in SEQ ID NO: 27; and the light chain variable region of the anti-PVRIG immunoglobulin comprises LCDR1 with the amino acid sequence as set forth in SEQ ID NO: 22, LCDR2 with the amino acid sequence as set forth in SEQ ID NO: 23, and LCDR3 with the amino acid sequence as set forth in SEQ ID NO: 24; and
the heavy chain variable region of the anti-TIGIT immunoglobulin comprises HCDR1 with the amino acid sequence as set forth in SEQ ID NO: 13, HCDR2 with the amino acid sequence as set forth in SEQ ID NO: 14, and HCDR3 with the amino acid sequence as set forth in SEQ ID NO: 15; and the light chain variable region of the anti-TIGIT immunoglobulin comprises LCDR1 with the amino acid sequence as set forth in SEQ ID NO: 16, LCDR2 with the amino acid sequence as set forth in SEQ ID NO: 17, and LCDR3 with the amino acid sequence as set forth in SEQ ID NO: 18; or
the heavy chain variable region of the anti-TIGIT immunoglobulin comprises HCDR1 with the amino acid sequence as set forth in SEQ ID NO: 19, HCDR2 with the amino acid sequence as set forth in SEQ ID NO: 20, and HCDR3 with the amino acid sequence as set forth in SEQ ID NO: 21; and the light chain variable region of the anti-TIGIT immunoglobulin comprises LCDR1 with the amino acid sequence as set forth in SEQ ID NO: 22, LCDR2 with the amino acid sequence as set forth in SEQ ID NO: 23, and LCDR3 with the amino acid sequence as set forth in SEQ ID NO: 24.

In some embodiments of the present invention, the bispecific antibody is provided, wherein:
the heavy chain variable region of the anti-PVRIG immunoglobulin or anti-PVRIG single-chain antibody has an amino acid sequence set forth in SEQ ID NO: 5; and
the light chain variable region of the anti-PVRIG immunoglobulin or anti-PVRIG single-chain antibody has an amino acid sequence set forth in SEQ ID NO: 4;
preferably, the glycine at position 44 of the heavy chain variable region of the anti-PVRIG immunoglobulin or anti-PVRIG single-chain antibody is replaced with a cysteine, and the glycine at position 100 of the light chain variable region of the anti-PVRIG immunoglobulin or anti-PVRIG single-chain antibody is replaced with a cysteine.

In some embodiments of the present invention, the bispecific antibody is provided, wherein:
the heavy chain variable region of the anti-TIGIT immunoglobulin or anti-TIGIT single-chain antibody has an amino acid sequence set forth in SEQ ID NO: 1; and the light chain variable region of the anti-TIGIT immunoglobulin or anti-TIGIT single-chain antibody has an amino acid sequence set forth in SEQ ID NO: 2;
   or,
the heavy chain variable region of the anti-TIGIT immunoglobulin or anti-TIGIT single-chain antibody has an amino acid sequence set forth in SEQ ID NO: 3; and the light chain variable region of the anti-TIGIT immunoglobulin or anti-TIGIT single-chain antibody has an amino acid sequence set forth in SEQ ID NO: 4;
preferably, the glycine at position 44 of the heavy chain variable region of the anti-TIGIT immunoglobulin or anti-TIGIT single-chain antibody is replaced with a cysteine, and the glycine at position 100 of the light chain variable region of the anti-TIGIT immunoglobulin or anti-TIGIT single-chain antibody is replaced with a cysteine.

In some embodiments of the present invention, the bispecific antibody is provided, wherein the first protein functional region and the second protein functional region are directly linked or linked via a linker;
preferably, the linker is (GGGGS)m, and m is a positive integer, such as 1, 2, 3, 4, 5, or 6;
preferably, the amino acid sequence of the linker is set forth in SEQ ID NO: 6.

In some embodiments of the present invention, the bispecific antibody is provided, wherein the number of the first protein functional region and the second protein functional region is independently 1, 2, or more than 2.

In some embodiments of the present invention, the bispecific antibody is provided, wherein,
the anti-TIGIT single-chain antibody (two molecules) is respectively linked to the C-terminus of the two heavy chains of the anti-PVRIG immunoglobulin; or
the anti-PVRIG single-chain antibody (two molecules) is respectively linked to the C-terminus of the two heavy chains of the anti-TIGIT immunoglobulin.

In some embodiments of the present invention, the bispecific antibody is provided, wherein,
the constant region of the anti-PVRIG immunoglobulin or anti-TIGIT immunoglobulin is derived from a human antibody;
preferably, the constant region is independently selected from the group consisting of the constant regions of human IgG1, IgG2, IgG3, or IgG4.

In some embodiments of the present invention, the bispecific antibody is provided, wherein,
the heavy chain constant region of the anti-PVRIG immunoglobulin or anti-TIGIT immunoglobulin is human Ig gamma-1 chain C region or human Ig gamma-4 chain C region, and the light chain constant region is human Ig kappa chain C region;
preferably, the heavy chain constant region of the anti-PVRIG immunoglobulin and anti-TIGIT immunoglobulin further comprises a L234A mutation and a L235A mutation according to the EU numbering system (abbreviated as LALA).

In the present invention, unless otherwise specified, the letter before the site represents the amino acid before mutation, and the letter after the site represents the amino acid after mutation.

In some embodiments of the present invention, the bispecific antibody is provided, which is a dimer or tetramer formed with a peptide chain having an amino acid sequence set forth in SEQ ID NO: 7 and a peptide chain having an amino acid sequence set forth in SEQ ID NO: 8, or a dimer or tetramer formed with a peptide chain having an amino acid sequence set forth in SEQ ID NO: 9 and a peptide chain having an amino acid sequence set forth in SEQ ID NO: 10.

Another aspect of the present invention relates to an isolated nucleic acid molecule, which encodes the bispecific antibody according to any embodiment of the present invention.

A further aspect of the present invention relates to a vector, which comprises the isolated nucleic acid molecule of the present invention.

A further aspect of the present invention relates to a host cell, which comprises the isolated nucleic acid molecule of the present invention, or the vector of the present invention.

A further aspect of the present invention relates a conjugate, which comprises a bispecific antibody and a conjugated moiety, wherein the bispecific antibody is the bispecific antibody according to any embodiment of the present invention, and the conjugated moiety is a detectable label; preferably, the conjugated moiety is a radioactive isotope, a fluorescent substance, a colored substance, or an enzyme.

Another aspect of the present invention relates to a kit, which comprises the bispecific antibody according to any embodiment of the present invention, or the conjugate of the present invention;
preferably, the kit further comprises a second antibody that is capable of specifically binding to the bispecific antibody; optionally, the second antibody further comprises a detectable label, such as a radioactive isotope, a fluorescent substance, a colored substance or an enzyme.

Another aspect of the present invention relates to a pharmaceutical composition, which comprises the bispecific antibody according to any embodiment of the present invention, and one or more pharmaceutically acceptable excipients;
preferably, the pharmaceutical composition further comprises at least one anti-PD-1 antibody;
preferably, the molar ratio of the bispecific antibody to the anti-PD-1 antibody is (1:5)- (5:1), for example, 1:5, 1:4, 1:3, 1:2, 1:1, 2:1, 3:1, 4:1, or 5:1; more preferably, it is 1:1.

In some embodiments of the present invention, the pharmaceutical composition is provided, wherein, the anti-PD-1 antibody, which comprises a heavy chain variable region and a light chain variable region. the heavy chain variable region comprises HCDR1 to HCDR3, and the light chain variable region comprises LCDR1 to LCDR3, wherein:
the heavy chain variable region of the anti-PD-1 antibody comprises HCDR1 with the amino acid sequence as set forth in SEQ ID NO: 36, HCDR2 with the amino acid sequence as set forth in SEQ ID NO: 37, and HCDR3 with the amino acid sequence as set forth in SEQ ID NO: 38; and the light chain variable region of the anti-PD-1 antibody comprises LCDR1 with the amino acid sequence as set forth in SEQ ID NO: 39, LCDR2 with the amino acid sequence as set forth in SEQ ID NO: 40, and LCDR3 with the amino acid sequence as set forth in SEQ ID NO: 41;
preferably, the heavy chain variable region of the anti-PD-1 antibody has an amino acid sequence as set forth in SEQ ID NO: 34, and the light chain variable region has an amino acid sequence as set forth in SEQ ID NO: 35.

In one or more embodiments of the present invention, the pharmaceutical composition is provided, wherein the unit dose of the pharmaceutical composition, calculated by the mass of the bispecific antibody contained therein, is 100mg-1000mg, 200mg-800mg, 200mg-500mg, 300mg-600mg, 400mg-500mg, or 450mg.

Another aspect of the present invention relates to a combination product, which comprises a first product and a second product in separate packages, wherein:
the first product comprises the bispecific antibody according to any embodiment of the present invention;
the second product comprises at least one anti-PD-1 antibody;
preferably, the first product and the second product further independently comprise one or more pharmaceutically acceptable excipients;
preferably, the combination product further comprises a package insert;

In some embodiments of the present invention, the combination product is provided, wherein the molar ratio of the bispecific antibody to the anti-PD-1 antibody is from (1:5) to (5:1), for example, 1:5, 1:4, 1:3, 1:2, 1:1, 2:1, 3:1, 4:1, or 5:1; more preferably 1:1.

In some embodiments of the present invention, the combination product is provided, wherein the anti-PD-1 antibody comprises a heavy chain variable region and a light chain variable region, the heavy chain variable region comprising HCDR1 to HCDR3, and the light chain variable region comprising LCDR1 to LCDR3, wherein,
the heavy chain variable region of the anti-PD-1 antibody comprises HCDR1 with the amino acid sequence as set forth in SEQ ID NO: 36, HCDR2 with the amino acid sequence as set forth in SEQ ID NO: 37, and HCDR3 with the amino acid sequence as set forth in SEQ ID NO: 38; and the light chain variable region of the anti-PD-1 antibody comprises LCDR1 with the amino acid sequence as set forth in SEQ ID NO: 39, LCDR2 with the amino acid sequence as set forth in SEQ ID NO: 40, and LCDR3 with the amino acid sequence as set forth in SEQ ID NO: 41;
preferably, the heavy chain variable region of the anti-PD-1 antibody has an amino acid sequence set forth in SEQ ID NO: 34, and the light chain variable region has an amino acid sequence set forth in SEQ ID NO: 35.

Another aspect of the present invention relates to a use of the bispecific antibody according to any embodiment of the present invention or the conjugate of the present invention in the manufacture of a medicament for the treatment or prevention of a tumor;
preferably, the tumor is one or more selected from the group consisting of colorectal cancer, melanoma, lung cancer, kidney cancer, endometrial cancer, breast cancer, skin cancer, ovarian cancer, stomach cancer, head and neck cancer, liver cancer, brain tumor, urinary tract cancer, bone tumor, bile duct cancer, rectal cancer, pancreatic cancer, cervical cancer, multiple myeloma, Hodgkin's lymphoma, non-Hodgkin's lymphoma, B-cell lymphoma, plasma cell cancer, prostate cancer, and testicular cancer;
preferably, the lung cancer is non-small cell lung cancer or small cell lung cancer.

The bispecific antibody according to any embodiment of the present invention, or the conjugate of the present invention, or the pharmaceutical composition according to any embodiment of the present invention for use in the treatment or prevention of a tumor;
preferably, the tumor is one or more selected from the group consisting of colorectal cancer, melanoma, lung cancer, kidney cancer, endometrial cancer, breast cancer, skin cancer, ovarian cancer, stomach cancer, head and neck cancer, liver cancer, brain tumor, urinary tract cancer, bone tumor, bile duct cancer, rectal cancer, pancreatic cancer, cervical cancer, multiple myeloma, Hodgkin's lymphoma, non-Hodgkin's lymphoma, B-cell lymphoma, plasma cell cancer, prostate cancer, and testicular cancer;
preferably, the lung cancer is non-small cell lung cancer or small cell lung cancer.

Another aspect of the present invention relates to a method for treating or preventing a tumor, comprising a step of administering to a subject in need thereof an effective amount of the bispecific antibody according to any embodiment of the present invention or the conjugate of the present invention, or the pharmaceutical composition according to any embodiment of the present invention;
preferably, the tumor is one or more selected from the group consisting of colorectal cancer, melanoma, lung cancer, kidney cancer, endometrial cancer, breast cancer, skin cancer, ovarian cancer, stomach cancer, head and neck cancer, liver cancer, brain tumor, urinary tract cancer, bone tumor, bile duct cancer, rectal cancer, pancreatic cancer, cervical cancer, multiple myeloma, Hodgkin's lymphoma, non-Hodgkin's lymphoma, B-cell lymphoma, plasma cell cancer, prostate cancer, and testicular cancer;
preferably, the lung cancer is non-small cell lung cancer or small cell lung cancer.

In one or more embodiments of the present invention, the method of treating and/or preventing the malignant tumor is provided, wherein,
the single dose of the bispecific antibody is 0.1 to 100mg, preferably 1 to10mg (e.g., 1 mg, 2 mg, 3 mg, 4 mg, 5 mg, 6 mg, 7 mg, 8 mg, 9 mg, or 10 mg), per kilogram of body weight,; alternatively, the single doseage of the bispecific antibody of the present invention is 10 to 1000mg per subject (e.g., approximately 100mg, approximately 150mg, approximately 200 mg, approximately 250 mg, approximately 300 mg, approximately 350 mg, approximately 400 mg, approximately 450 mg, approximately 500 mg, approximately 600 mg, approximately 700 mg, approximately 800 mg, approximately 900 mg, or approximately 1000 mg), preferably 50-500mg, 100-400mg, 150-300mg, 150-250mg, or 200mg, per kilogram of body weight;
preferably, the administering is performed every 3 days, 4 days, 5 days, 6 days, 10 days, 1 week, 2 weeks, or 3 weeks;
preferably, the administering is performed by the manner of intravenous drip or intravenous injection.

In some regimens, the administration of the bispecific antibody is in cycles of 2 weeks (14 days) or 3 weeks (21 days), preferably with the anti-PVRIG antibody administered intravenously on the first day (D1) of each cycle. For example, the bispecific antibody is administered at a frequency of once every two weeks (q2w) or once every three weeks (q3w).

In the present invention, unless otherwise stated, scientific and technical terms used herein have the meanings commonly understood by those skilled in the art. Moreover, the procedures of cell culture, molecular genetics, nucleic acid chemistry, and immunology laboratory used herein are routine procedures widely used in the corresponding fields. Meanwhile, in order to better understand the present invention, definitions and explanations of relevant terms are provided below.

As used herein, when referring to the amino acid sequence of PVRIG (NCBI GenBank ID: NP_076975.2), it includes the full-length PVRIG protein or its extracellular domain; it also includes fusion proteins of PVRIG, such as a fragment fused to a mouse or human IgG Fc protein fragment (mFc or hFc). However, those skilled in the art understand that in the amino acid sequence of PVRIG protein, a mutation or variation (including but not limited to substitution, deletion and/or addition) can occur naturally or be artificially introduced without affecting its biological function. Therefore, in the present invention, the term " PVRIG protein" or "PVRIG" shall include all such sequences, including the sequences shown and natural or artificial variants thereof. Moreover, when describing a sequence fragment of PVRIG protein, it includes not only the sequence fragment, but also the corresponding sequence fragment in its natural or artificial variants.

As used herein, when referring to the amino acid sequence of TIGIT (NCBI GenBank ID: NP_776160.2), it includes the full-length TIGIT protein or its functional fragment; it also includes fusion proteins of TIGIT, such as a fragment fused to a mouse or human IgG Fc protein fragment (mFc or hFc). However, those skilled in the art understand that in the amino acid sequence of TIGIT protein, a mutation or variation (including but not limited to substitution, deletion and/or addition) can occur naturally or be artificially introduced without affecting its biological function. Therefore, in the present invention, the term " TIGITprotein" or "TIGIT" shall include all such sequences, including the sequences shown and natural or artificial variants thereof. Moreover, when describing a sequence fragment of TIGIT protein, it includes not only the sequence fragment, but also the corresponding sequence fragment in its natural or artificial variants.

As used herein, the term "EC₅₀" refers to a concentration for 50% of maximal effect, which refers to a concentration that causes 50% of maximum effect.

As used herein, the term "antibody" refers to an immunoglobulin molecule usually composed of two pairs of polypeptide chains, each pair having a "light" (L) chain and a "heavy" (H) chain. Antibody light chains can be classified into κ and λ light chains. Heavy chains can be classified as µ, δ, γ, α, or ε, and define the antibody's isotype as IgM, IgD, IgG, IgA, and IgE, respectively. Within the light and heavy chains, the variable and constant regions are connected by a "J" region of approximately 12 or more amino acids, and the heavy chain further contains a "D" region of approximately 3 or more amino acids. Each heavy chain consists of a heavy chain variable region (VH) and a heavy chain constant region (CH). The heavy chain constant region consists of 3 domains (CH1, CH2 and CH3). Each light chain consists of a light chain variable region (VL) and a light chain constant region (CL). The light chain constant region consists of one domain, CL. The constant region of antibody may mediate the binding of immunoglobulin to host tissues or factors, including various cells (e.g., effector cells) of immune system and first component (Clq) of classical complement system. The VH and VL regions can also be subdivided into highly variable regions called complementarity determining regions (CDRs), interspersed therewith more conservative regions called framework regions (FRs). Each VH and VL consists of 3 CDRs and 4 FRs arranged from the amino terminus to the carboxyl terminus in the following order: FR1, CDR1, FR2, CDR2, FR3, CDR3 and FR4. The variable regions (VH and VL) of each heavy chain/light chain pair respectively form an antibody binding site. The assignment of amino acids to regions or domains follows the definition of Bethesda M.d., Kabat Sequences of Proteins of Immunological Interest (National Institutes of Health, (1987 and 1991)), or Chothia & Lesk J. Mol. Biol. 1987; 196: 901-917; Chothia et al, Nature, 1989; 342: 878-883; or the IMGT numbering system, see, the definition of Ehrenmann F, Kaas Q, Lefranc M P. IMGT/3Dstructure-DB and IMGT/DomainGapAlign: a database and a tool for immunoglobulins or antibodies, T cell receptors, MHC, IgSF and MhcSF [J]. Nucleic acids research, 2009; 38(suppl_1): of D301-D307. The term "antibody" is not limited to any particular method of producing antibody, which includes, for example, recombinant antibodies, monoclonal antibodies, and polyclonal antibodies. Antibodies may be of different isotypes, for example, IgG (e.g., IgGl, IgG2, IgG3 or IgG4 subtype), IgA1, IgA2, IgD, IgE or IgM antibody.

As used herein, the term "antigen-binding fragment", also known as the "antigen-binding portion", refers to a polypeptide comprising the fragment of a full-length antibody, which maintains the ability to specifically bind to the same antigen to which the full-length antibody binds, and/or competes with the full-length antibody for the specific binding to the antigen. See generally, Fundamental Immunology, Ch. 7 (Paul, W., ed., 2nd edition, Raven Press, N.Y. (1989), which is incorporated herein by reference in its entirety for all purposes. The antigen-binding fragment of the antibody can be produced by recombinant DNA technology or by enzymatic or chemical cleavage of an intact antibody. In some cases, the antigen-binding fragment includes Fab, Fab', F(ab')2, Fd, Fv, dAb, and complementarity determining region (CDR) fragments, single chain antibody fragments (e.g., scFv), chimeric antibodies, diabodies, and polypeptides comprising at least a portion of the antibody sufficient to impart specific antigen binding ability to them.

As used herein, the term "Fd fragment" refers to an antibody fragment consisting of V_{H} and C_{H}1 domains; the term "Fv fragment" refers to an antibody fragment consisting of the V_{L} and VH domains of a single arm of an antibody; the term "dAb fragment" refers to an antibody fragment consisting of a VH domain (Ward et al., Nature 341:544-546 (1989)); the term "Fab fragment" refers to an antibody fragment consisting of V_{L}, V_{H}, C_{L} and CH1 domains; and the term "F(ab')2 fragment" refers to an antibody fragment comprising two Fab fragments linked by the disulfide bridge on a hinge region.

In some cases, the antigen-binding fragment of the antibody is a single chain antibody (e.g., scFv) in which the V_{L} and V_{H} domains are paired to form a monovalent molecule via a linker that enables them to produce a single polypeptide chain (see, e.g., Bird et al., Science 242:423-426 (1988) and Huston et al., Proc. Natl. Acad. Sci. USA 85:5879-5883 (1988)). Such scFv molecules may have a general structure: NH₂-V_{L}-linker-V_{H}-COOH or NH₂-V_{H}-linker-V_{L}-COOH. An appropriate linker in prior art consists of GGGGS amino acid sequence repeats or a variant thereof. For example, a linker having the amino acid sequence (GGGGS)₄ can be used, but variants thereof can also be used (Holliger et al. (1993), Proc. Natl. Acad. Sci. USA 90: 6444-6448). Other linkers that can be used in the present invention are described by Alfthan et al. (1995), Protein Eng. 8:725-731; Choi et al. (2001), Eur. J. Immunol. 31: 94-106; Hu et al. (1996), Cancer Res. 56:3055-3061; Kipriyanov et al. (1999), J. Mol. Biol. 293:41-56; and Roovers et al. (2001), Cancer Immunol.

In some cases, the antigen-binding fragment of the antibody is a diabody, that is, a bivalent antibody, in which the V_{H} and V_{L} domains are expressed on a single polypeptide chain. However, the linker used is too short to allow the pairing of the two domains on one chain, thereby the domains are forced to pair with the complementary domains on another chain and two antigen-binding sites are generated (see, e.g., Holliger P. et al., Proc. Natl. Acad. Sci. USA 90:6444-6448 (1993), and Poljak R. J. et al., Structure 2: 1121-1123 (1994)).

In other cases, the antigen-binding fragment of the antibody is a "bispecific antibody", which refers to a conjugate formed from a primary antibody (fragment) and a secondary antibody (fragment) or antibody analog via a linker; the methods of conjugation include, but are not limited to, chemical reaction, gene fusion, and enzyme catalysis. The antigen-binding fragment of the antibody may be a "multispecific antibody" including, for example, a trispecific antibody and a tetraspecific antibody, the former being an antibody with three different kinds of antigen-binding specificity, and the latter being an antibody with four different kinds of antigen-binding specificity. For example, a designed ankyrin repeat protein (DARPin) is linked to an IgG antibody, a scFv-Fc antibody fragment or combinations thereof, such as CN104341529A. An anti-IL-17a fynomer binds to an anti-IL-6R antibody, such as WO2015141862A1.

Antigen-binding fragments of antibodies (e.g., the antibody fragments described above) can be obtained from a given antibody (e.g., monoclonal antibody ADI-56127, ADI-55796 or ADI-55812 provided in the present invention) using conventional techniques known to those skilled in the art (e.g., recombinant DNA technology or enzymatic or chemical cleavage), and the antigen-binding fragments of antibodies are screened for specificity in the same manner as for intact antibodies.

The terms "mcAb" and "monoclonal antibody" refer to an antibody or a fragment of antibody from a group of highly homologous antibody molecules, that is, a group of identical antibody molecules except for natural mutations that may occur spontaneously. Monoclonal antibody is highly specific for a single epitope on an antigen. Polyclonal antibody is relative to monoclonal antibody, which usually comprises at least two or more different antibodies, and these different antibodies usually recognize different epitopes on an antigen. Monoclonal antibodies can usually be obtained using the hybridoma technology that was first reported by Kohler et al. (Köhler G, Milstein C. Continuous cultures of fused cells secreting antibody of predefined specificity[J]. nature, 1975; 256(5517): 495), but also can be obtained using the recombinant DNA technology (e.g., see, U.S. Patent 4,816,567).

As used herein, the term "humanized antibody" refers to an antibody or antibody fragment obtained when all or a part of CDRs of a human immunoglobulin (receptor antibody) are replaced by the CDRs of a non-human antibody (donor antibody), wherein the donor antibody may be a non-human (e.g., mouse, rat, or rabbit) antibody having expected specificity, affinity, or reactivity. In addition, some amino acid residues in the framework regions (FRs) of the receptor antibody can also be replaced by the amino acid residues of corresponding non-human antibodies or by the amino acid residues of other antibodies to further improve or optimize the performance of the antibody. For more details on humanized antibodies, see, e.g., Jones et al., Nature, 1986; 321:522-525; Reichmann et al., Nature, 1988; 332:323-329; Presta, Curr. Op. Struct. Biol. 1992; 2:593-596; and Clark, Immunol. Today, 2000; 21:397-402.

As used herein, the term "isolated" or "being isolated" refers to being obtained from the natural state by artificial means. If an "isolated" substance or ingredient occurs in nature, it may be due to a change in its natural environment, or the substance has been separated from its natural environment, or both. For example, a certain unisolated polynucleotide or polypeptide naturally exists in a living animal, and the same polynucleotide or polypeptide with high purity isolated from this natural state is called "isolated". The term "isolated" or "being isolated" does not exclude the admixture of artificial or synthetic substances, nor does it exclude the presence of other impure substances that do not affect the activity of the substance.

As used herein, the term "vector" refers to a nucleic acid delivery vehicle into which a polynucleotide can be inserted. When a vector can express the protein encoded by the inserted polynucleotide, the vector is called an expression vector. The vector can be introduced into a host cell through transformation, transduction or transfection, so that the genetic material elements it carries can be expressed in the host cell. Vectors are well known to those skilled in the art, including but not limited to: plasmids; phagemids; cosmids; artificial chromosomes, such as yeast artificial chromosomes (YAC), bacterial artificial chromosomes (BAC) or P1-derived artificial chromosomes (PAC); phages such as λ phage or M13 phage, and animal viruses, etc. Animal viruses that can be used as vectors include, but are not limited to, retrovirus (including lentivirus), adenovirus, adeno-associated virus, herpesvirus (e.g., herpes simplex virus), poxvirus, baculovirus, papillomavirus, papovavirus (e.g., SV40). A vector may comprise a variety of expression-controlling elements, including, but not limited to, promoter sequence, transcription initiation sequence, enhancer sequence, selection element, and reporter gene. In addition, the vector may also comprise an origin of replication site.

As used herein, the term "host cell" refers to a cell that can be used to introduce a vector, which includes, but is not limited to, prokaryotic cell such as *Escherichia coli* or *Bacillus subtilis,* fungal cell such as yeast cell or *Aspergillus,* etc., insect cell such as *S2 Drosophila* cell or Sf9, or animal cell such as fibroblast, CHO cell, COS cell, NSO cell, HeLa cell, GS cell, BHK cell, HEK 293 cell or human cell.

As used herein, the term "specific binding" refers to a non-random binding reaction between two molecules, such as the reaction between an antibody and an antigen to which it targets. In certain embodiments, an antibody that specifically binds to an antigen (or an antibody specific for an antigen) refers to an antibody that binds to the antigen with an affinity (K_{D}) of less than about 10⁻⁵ M, such as less than about 10⁻⁶ M, 10⁻⁷ M, 10⁻⁸ M, 10⁻⁹ M, or 10⁻¹⁰ M or less.

As used herein, the term "K_{D}" refers to the dissociation equilibrium constant of a specific antibody-antigen interaction, which is used to describe the binding affinity between the antibody and the antigen. The smaller the equilibrium dissociation constant, the tighter the antibody-antigen binding, and the higher the affinity between the antibody and the antigen. Typically, an antibody binds to an antigen (e.g., TIGIT protein) with a dissociation equilibrium constant (K_{D}) of less than about 10⁻⁵ M, such as less than about 10⁻⁶ M, 10⁻⁷ M, 10⁻⁸ M, 10⁻⁹ M, or 10⁻¹⁰ M or less. K_{D} can be determined using methods known to those skilled in the art, such as using a Fortebio molecular interaction instrument.

As used herein, the terms "monoclonal antibody" and "McAb" have the same meaning and are used interchangeably; the terms "polyclonal antibody" and "PcAb" have the same meaning and are used interchangeably. And in the present invention, amino acids are generally represented by one-letter and three-letter abbreviations well known in the art. For example, alanine can be represented by A or Ala.

As used herein, the term "pharmaceutically acceptable excipient" refers to a carrier and/or excipient that is pharmacologically and/or physiologically compatible with a subject and an active ingredient. It is well known in the art (see, for example, Remington's Pharmaceutical Sciences. Edited by Gennaro AR, 19th ed. Pennsylvania: Mack Publishing Company, 1995), and includes, but is not limited to: pH adjuster, surfactant, adjuvant, ionic strength enhancer. For example, the pH adjuster includes but is not limited to phosphate buffer; the surfactant includes but is not limited to cationic, anionic or nonionic surfactant such as Tween-80; and the ionic strength enhancer includes but is not limited to sodium chloride.

As used herein, the term "effective amount" refers to an amount sufficient to obtain, at least partially, the desired effect. For example, a prophylactically effective amount is an amount that is sufficient to prevent, arrest, or delay the occurrence of a disease (e.g., a tumor); a therapeutically effective amount is an amount that is sufficient to cure or at least partially prevent a disease and its complications in a patient who already has the disease.

As used herein, the terms "hybridoma" and "hybridoma cell line" are interchangeable, and when referring to "hybridoma" and "hybridoma cell line," it also includes subclones and progeny cells of the hybridoma.

In the present invention, unless otherwise specified, the terms "first" (e.g., first product) and "second" (e.g., second product) are used for distinction or clarity of expression and do not imply any typical order of precedence.

### Beneficial effects of the present invention

The present invention achieves one or more of the following technical effects as described in items (1) to (4):
(1) The bispecific antibody of the present invention is capable of binding to TIGIT and PVRIG with high affinity.
(2) The bispecific antibody of the present invention is likely to have a synergistic effect with anti-PD-1 antibodies or anti-PD-L1 antibodies.
(3) The antibody of the present invention is effective in treating and/or preventing tumors, such as colorectal cancer, melanoma, lung cancer, kidney cancer, endometrial cancer, breast cancer, skin cancer, ovarian cancer, stomach cancer, head and neck cancer, liver cancer, brain tumors, urothelial cancer, bone tumors, bile duct cancer, rectal cancer, pancreatic cancer, cervical cancer, multiple myeloma, Hodgkin's lymphoma, non-Hodgkin's lymphoma, B-cell lymphoma, plasma cell cancer, prostate cancer, and testicular cancer, among others.
(4) The bispecific antibody of the present invention has a low level of toxicity and side effects.

### Brief Description of the Drawings

Fig. 1A to 1C shows the schematic structures of the bispecific antibody of the present invention.
Fig. 2 shows the binding curve of the bispecific antibody of the present invention to human TIGIT overexpressed on CHO cells.
Fig. 3 shows the binding curve of the bispecific antibody of the present invention to cynomolgus TIGIT overexpressed on CHO cells.
Fig. 4 shows the binding curve of the bispecific antibody of the present invention to mouse TIGIT overexpressed on CHO cells.
Fig. 5 shows the curve of the bispecific antibody of the present invention blocking the binding of human CD155 to human TIGIT overexpressed on CHO cells.
Fig. 6 shows the curve of the bispecific antibody of the present invention blocking the binding of mouse CD155 to mouse TIGIT overexpressed on CHO cells.
Fig. 7 shows the binding curve of the bispecific antibody of the present invention to human PVRIG overexpressed on CHO cells.
Fig. 8 shows the binding curve of the bispecific antibody of the present invention to cynomolgus PVRIG overexpressed on CHO cells.
Fig. 9 shows the binding curve of the bispecific antibody of the present invention to mouse PVRIG overexpressed on CHO cells.
Fig. 10 shows the curve of the bispecific antibody of the present invention blocking the binding of human PVRIG to human CD112 overexpressed on CHO cells.
Fig. 11 shows the curve of the bispecific antibody of the present invention blocking the binding of mouse CD112 to mouse PVRIG protein.
Fig. 12 shows the curve of the bispecific antibody of the present invention co-binding to human PVRIG and human TIGIT protein.
Fig. 13 shows the curve of the bispecific antibody of the present invention blocking PVRIG/CD112 and TIGIT/CD155 signaling pathways.
Figs. 14A to 14B show the curve of the bispecific antibody of the present invention in combination with anti-PD-1/L1 antibodies blocking PVRIG/CD112, TIGIT/CD155 and PD-1/PD-L1 signaling pathways.
Fig. 15 shows the efficacy of the bispecific antibody of the present invention in the B-NDG mouse model inoculated with mixed A375 and human PBMC.
Figs. 16A to 16B show the efficacy of the bispecific antibody of the present invention in combination with anti-PD-1 antibody in the B-NDG mouse model inoculated with mixed A375 and human PBMC.
Fig. 17 shows the in vivo half-life curve of the bispecific antibody of the present invention in mice.
Fig. 18 shows the binding curve of the anti-TIGIT antibody of the present invention to human TIGIT overexpressed on CHO cells.
Fig. 19 shows the binding curve of the anti-TIGIT antibody of the present invention to cynomolgus TIGIT overexpressed on CHO cells.
Fig. 20 shows the binding curve of the anti-TIGIT antibody of the present invention to mouse TIGIT overexpressed on CHO cells.
Fig. 21 shows the curve of the anti-TIGIT antibody of the present invention blocking the binding of human CD155 to human TIGIT overexpressed on CHO cells.
Fig. 22 shows the curve of the anti-TIGIT antibody of the present invention blocking the binding of mouse CD155 to mouse TIGIT overexpressed on CHO cells.
Fig. 23 shows the binding curve of the anti-TIGIT antibody of the present invention to TIGIT on activated human primary T cells.
Fig. 24 shows the binding curve of the anti-PVRIG antibody of the present invention to human PVRIG overexpressed on CHO cells.
Fig. 25 shows the binding curve of the anti-PVRIG antibody of the present invention to cynomolgus PVRIG overexpressed on CHO cells.
Fig. 26 shows the binding curve of the anti-PVRIG antibody of the present invention to mouse PVRIG overexpressed on CHO cells.
Fig. 27 shows the curve of the anti-PVRIG antibody of the present invention blocking the binding of human PVRIG to human CD112 overexpressed on CHO cells.
Fig. 28 shows the curve of the anti-PVRIG antibody of the present invention blocking the binding of mouse CD112 to mouse PVRIG protein.
Fig. 29 shows the binding curve of the anti-PVRIG antibody of the present invention to PVRIG on activated human primary T cells.
Fig. 30 shows the drug efficacy of the anti-PVRIG antibody of the present invention in the NDG mouse model inoculated with mixed A375 and human PBMC.

### Specific Models for Carrying Out the present invention

The present invention will be further described below in conjunction with specific examples. It should be understood that these examples are only used to illustrate the present invention and are not intended to limit the scope of the present invention. The experimental methods without specifying specific conditions in the following examples usually followed conventional conditions, such as the conditions described in Sambrook et al., Molecular Cloning: Laboratory Manual (New York: Cold Spring Harbor Laboratory Press, 1989), or the conditions recommended by the manufacturer. Unless otherwise stated, percentages and parts are by weight.

Antibody Atezolizumab: anti-PD-L1 monoclonal antibody, trade name Tecentriq, Roche.

Antibody Pembrolizumab: anti-PD-1 monoclonal antibody, trade name Keytruda, Merck.

The following control antibodies used in the examples were expressed and purified from HEK293 cells:
COM701 is an anti-human PVRIG antibody from Compugen expressed by HEK293 cells with a light chain variable region sequence and a heavy chain variable region sequence corresponding to SEQ ID NO: 1372, SEQ ID NO: 1380 in US Patent US10227408B2.
Mab46 is an anti-human PVRIG antibody from Surface Oncology expressed by HEK293 cells with light chain variable region and heavy chain variable region sequences corresponding to SEQ ID NO: 912, SEQ ID NO: 918 in US Patent Publication US20200040081A1.
Tiragolumab is an anti-human TIGIT antibody from Genentech expressed by HEK293 cells with light and heavy chain sequences derived from WHO Drug Information, Vol. 31, No. 2, 2017, Proposed INN. List 117, CAS No. 1918185-84-8.

### Example 1: Design and preparation of anti-TIGIT-anti-PVRIG bispecific antibody

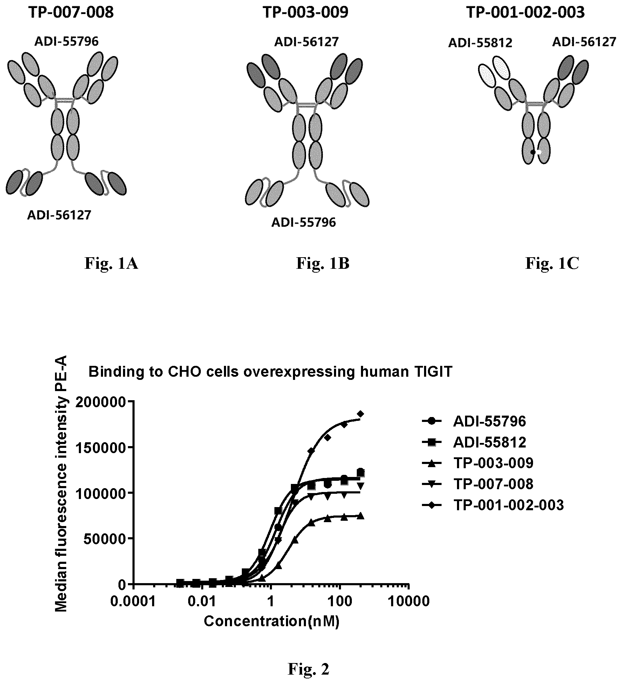

In this example, the scFv of the anti-PVRIG monoclonal antibody ADI-56127 was tandemly linked to the C-terminus of the heavy chain of the anti-TIGIT monoclonal antibody ADI-55796 via a GGGGSGGGGSG linker (SEQ ID NO: 44) (other linkers could also be used such as GGGGSGGGGS, SEQ ID NO: 6) by whole gene synthesis (Fig. 1A). The scFv of the anti-TIGIT monoclonal antibody ADI-55796 was tandemly linked to the C-terminus of the heavy chain of the anti-PVRIG monoclonal antibody ADI-56127 via a GGGGSGGGGSG linker (SEQ ID NO: 44) (other linkers could also be used such as GGGGSGGGGS, SEQ ID NO: 6) by whole gene synthesis (Fig. 1B). The heavy chain of the anti-TIGIT monoclonal antibody ADI-55812 (mutated in the CH3 region to form the "Knob" structure) and the heavy chain of the anti-PVRIG monoclonal antibody ADI-56127 (mutated in the CH3 region to form the "Hole" structure) were assembled to to form a "1+1" molecular structure by the "Knob into hole" method (Fig. 1C). Three different structures of anti-TIGIT/PVRIG bispecific antibodies, named TP-007-008, TP-003-009, and TP-001-002-003, were formed, and their schematic structures were shown in Fig. 1A to Fig. 1C. The scFv of the anti-TIGIT antibody and the anti-PVRIG antibody were composed of a "VH-linker-VL" linkage, and the flexible linker peptide between VH and VL was GGGGSGGGGSGGGGSGGGGSG (SEQ ID NO: 45) (other flexible linker peptides could also be used such as GGGGSGGGGSGGGGSGGGGS, SEQ ID NO: 46). The glycine at position 44 of the heavy chain variable region and position 100 of the light chain variable region of the anti-TIGIT monoclonal antibody ADI-55796 and the anti-PVRIG monoclonal antibody ADI-56127 were mutated to cysteine to improve the stability of scFv, respectively. All three bispecific antibodies were of the human IgG1 subtype. The sequences of the anti-TIGIT monoclonal antibodies ADI-55796 and ADI-55812, the anti-PVRIG monoclonal antibody ADI-56127, as well as the bispecific antibodies formed, were shown in Table 1 below.

**Table 1: Sequences of the anti-TIGIT-anti-PVRIG bispecific antibody and corresponding single-terminal antibody**

| Name or meaning | Sequences | SEQ ID NO: |
|---|---|---|
| Heavy chain variable region of the anti-TIGIT monoclonal antibody ADI-55796 | QLQLQESGPGLVKPAETLSLTCTVSGGSISSYD HYWTWIRQPPGKGLEWIGTVYYSGSTFHNPSL KSRVTIPVDTSKNQFSLKLSSVTAADTAVYYC ARVGPDVSHPPFDYWGQGTLVTVSS | 1 |
| Light chain variable region of the anti-TIGIT monoclonal antibody ADI-55796 | DIQMTQSPSSLSASVGDRVTITCRASQSISSYLN WYQQKPGKAPKLLIYAASSLQSGVPSRFSGSG SGTDFTLTISSLQPEDFATYYCQQSYSTPITFGG GTKVEIK | 2 |
| Heavy chain variable region of the anti-TIGIT monoclonal antibody ADI-55812 | QVQLVQSGAEVKKPGASVKVSCKASGYAFTG YYMHWVRQAPGQGLEWMGSIIPFSGEANYAQ KFQGRVTMTRDTSISTAYMELSRLRSDDTAVY YCARGPGSLDRLWYYYYGMDVWGQGTTVTV | 3 |
| | SS | |
| Light chain variable region of the anti-TIGIT monoclonal antibody ADI-55812 | EIVLTQSPGTLSLSPGERATLSCRASQSVSSSYL AWYQQKPGQAPRLLIYGASSRATGIPDRFSGS GSGTDFTLTISRLEPEDFAVYYCQQYGSSPITF GGGTKVEIK (identical to the sequences of the light chain variable region of the anti-PVRIG monoclonal antibody ADI-56127) | 4 |
| Heavy chain variable region of the anti-PVRIG monoclonal antibody ADI-56127 | QVQLVQSGAEVKKPGSSVKVSCKASGGTESV DAISWVRQAPGQGLEWMGDIIPFFDTDYAQKF QGRVTITADESTSTAYMELSSLRSEDTAVYYC AREGGTSWTHFFDLWGRGTLVTVSS | 5 |
| Light chain variable region of the anti-PVRIG monoclonal antibody ADI-56127 | EIVLTQSPGTLSLSPGERATLSCRASQSVSSSYL AWYQQKPGQAPRLLIYGASSRATGIPDRFSGS GSGTDFTLTISRLEPEDFAVYYCQQYGSSPITF GGGTKVEIK (identical to the sequences of the light chain variable region of the anti-TIGIT monoclonal antibody ADI-55812) | 4 |
| G₄S linker | GGGGSGGGGS | 6 |
| Peptide # 1 of the anti-TIGIT-anti-PVRIG bispecific antibody TP-007-008 | QLQLQESGPGLVKPAETLSLTCTVSGGSISSYD HYWTWIRQPPGKGLEWIGTVYYSGSTFHNPSL KSRVTIPVDTSKNQFSLKLSSVTAADTAVYYC ARVGPDVSHPPFDYWGQGTLVTVSSASTKGPS VFPLAPSSKSTSGGTAALGCLVKDYFPEPVTVS WNSGALTSGVHTFPAVLQSSGLYSLSSVVTVP SSSLGTQTYICNVNHKPSNTKVDKKVEPKSCD KTHTCPPCPAPELLGGPSVFLFPPKPKDTLMIS RTPEVTCVVVDVSHEDPEVKFNWYVDGVEVH | 7 |
| | NAKTKPREEQYNSTYRVVSVLTVLHQDWLNG KEYKCKVSNKALPAPIEKTISKAKGQPREPQV YTLPPSREEMTKNQVSLTCLVKGFYPSDIAVE WESNGQPENNYKTTPPVLDSDGSFFLYSKLTV DKSRWQQGNVFSCSVMHEALHNHYTQKSLSL SPGGGGGSGGGGSGQVQLVQSGAEVKKPGSS VKVSCKASGGTESVDAISWVRQAPGQCLEWM GDIIPFFDTDYAQKFQGRVTITADESTSTAYME LSSLRSEDTAVYYCAREGGTSWTHFFDLWGR GTLVTVSSGGGGSGGGGSGGGGSGGGGSGEIV LTQSPGTLSLSPGERATLSCRASQSVSSSYLAW YQQKPGQAPRLLIYGASSRATGIPDRFSGSGSG TDFTLTISRLEPEDFAVYYCQQYGSSPITFGCG TKVEIK | |
| Peptide # 2 of the anti- TIGIT-anti-PVRIG bispecific antibody TP-007-008 (the kappa light chain of the anti-TIGIT antibody ADI-55796) | DIQMTQSPSSLSASVGDRVTITCRASQSISSYLN WYQQKPGKAPKLLIYAASSLQSGVPSRFSGSG SGTDFTLTISSLQPEDFATYYCQQSYSTPITFGG GTKVEIKRTVAAPSVFIFPPSDEQLKSGTASVV CLLNNFYPREAKVQWKVDNALQSGNSQESVT EQDSKDSTYSLSSTLTLSKADYEKHKVYACEV THQGLSSPVTKSFNRGEC | 8 |
| Peptide # 1 of the anti- TIGIT-anti-PVRIG bispecific antibody TP-003-009 | QVQLVQSGAEVKKPGSSVKVSCKASGGTESV DAISWVRQAPGQGLEWMGDIIPFFDTDYAQKF QGRVTITADESTSTAYMELSSLRSEDTAVYYC AREGGTSWTHFFDLWGRGTLVTVSS ASTKGPSVFPLAPSSKSTSGGTAALGCLVKDY FPEPVTVSWNSGALTSGVHTFPAVLQSSGLYS LSSVVTVPSSSLGTQTYICNVNHKPSNTKVDK | 9 |
| | KVEPKSCDKTHTCPPCPAPELLGGPSVFLFPPK PKDTLMISRTPEVTCVVVDVSHEDPEVKFNWY VDGVEVHNAKTKPREEQYNSTYRVVSVLTVL HQDWLNGKEYKCKVSNKALPAPIEKTISKAKG QPREPQVYTLPPSREEMTKNQVSLTCLVKGFY PSDIAVEWESNGQPENNYKTTPPVLDSDGSFFL YSKLTVDKSRWQQGNVFSCSVMHEALHNHYT QKSLSLSPGGGGGSGGGGSGQLQLQESGPGLV KPAETLSLTCTVSGGSISSYDHYWTWIRQPPG KCLEWIGTVYYSGSTFHNPSLKSRVTIPVDTSK NQFSLKLSSVTAADTAVYYCARVGPDVSHPPF DYWGQGTLVTVSSGGGGSGGGGSGGGGSGG GGSGDIQMTQSPSSLSASVGDRVTITCRASQSI SSYLNWYQQKPGKAPKLLIYAASSLQSGVPSR FSGSGSGTDFTLTISSLQPEDFATYYCQQSYSTP ITFGCGTKVEIK | |
| Peptide # 2 of the anti- TIGIT-anti-PVRIG bispecific antibody TP-003-009 (the kappa light chain of the anti-PVRIG antibody ADI-56127) | EIVLTQSPGTLSLSPGERATLSCRASQSVSSSYL AWYQQKPGQAPRLLIYGASSRATGIPDRFSGS GSGTDFTLTISRLEPEDFAVYYCQQYGSSPITF GGGTKVEIKRTVAAPSVFIFPPSDEQLKSGTAS VVCLLNNFYPREAKVQWKVDNALQSGNSQES VTEQDSKDSTYSLSSTLTLSKADYEKHKVYAC EVTHQGLSSPVTKSFNRGEC | 10 |
| Peptide # 1 of the anti- TIGIT-anti-PVRIG bispecific antibody TP-001-002-003 | QVQLVQSGAEVKKPGASVKVSCKASGYAFTG YYMHWVRQAPGQGLEWMGSIIPFSGEANYAQ KFQGRVTMTRDTSISTAYMELSRLRSDDTAVY YCARGPGSLDRLWYYYYGMDVWGQGTTVTV SSASTKGPSVFPLAPSSKSTSGGTAALGCLVKD | 11 |
| | YFPEPVTVSWNSGALTSGVHTFPAVLQSSGLY SLSSVVTVPSSSLGTQTYICNVNHKPSNTKVDK KVEPKSCDKTHTCPPCPAPELLGGPSVFLFPPK PKDTLMISRTPEVTCVVVDVSHEDPEVKFNWY VDGVEVHNAKTKPREEQYNSTYRVVSVLTVL HQDWLNGKEYKCKVSNKALPAPIEKTISKAKG QPREPQVYTLPPCRDELTKNQVSLWCLVKGFY PSDIAVEWESNGQPENNYKTTPPVLDSDGSFFL YSKLTVDKSRWQQGNVFSCSVMHEALHNHYT QKSLSLSPG | |
| Peptide # 2 of the anti-TIGIT-anti-PVRIG bispecific antibody TP-001-002-003 | QVQLVQSGAEVKKPGSSVKVSCKASGGTESV DAISWVRQAPGQGLEWMGDIIPFFDTDYAQKF QGRVTITADESTSTAYMELSSLRSEDTAVYYC AREGGTSWTHFFDLWGRGTLVTVSS ASTKGPSVFPLAPSSKSTSGGTAALGCLVKDY FPEPVTVSWNSGALTSGVHTFPAVLQSSGLYS LSSVVTVPSSSLGTQTYICNVNHKPSNTKVDK KVEPKSCDKTHTCPPCPAPELLGGPSVFLFPPK PKDTLMISRTPEVTCVVVDVSHEDPEVKFNWY VDGVEVHNAKTKPREEQYNSTYRVVSVLTVL HQDWLNGKEYKCKVSNKALPAPIEKTISKAKG QPREPQVCTLPPSRDELTKNQVSLSCAVKGFY PSDIAVEWESNGQPENNYKTTPPVLDSDGSFFL VSKLTVDKSRWQQGNVFSCSVMHEALHNHYT QKSLSLSPG | 12 |
| Peptide # 3 of the anti-TIGIT-anti-PVRIG bispecific antibody TP-001-002-003 (the kappa light chain of the anti-TIGIT antibody ADI-55812) | Identical to the sequences of the peptide # 2 of the anti-TIGIT-anti-PVRIG bispecific antibody TP-003-009 | 10 |
| HCDR1 of the heavy chain variable region of the anti-TIGIT monoclonal antibody ADI-55796 | GSISSYDHYWT | 13 |
| HCDR2 of the heavy chain variable region of the anti-TIGIT monoclonal antibody ADI-55796 | TVYYSGSTFHNPSLKS | 14 |
| HCDR3 of the heavy chain variable region of the anti-TIGIT monoclonal antibody ADI-55796 | ARVGPDVSHPPFDY | 15 |
| LCDR1 of the light chain variable region of the anti-TIGIT monoclonal antibody ADI-55796 | RASQSISSYLN | 16 |
| LCDR2 of the light chain variable region of the anti-TIGIT monoclonal antibody ADI-55796 | AASSLQS | 17 |
| LCDR3 of the light chain variable region of the anti-TIGIT monoclonal antibody ADI-55796 | QQSYSTPIT | 18 |
| HCDR1 of the heavy chain variable region of the anti-TIGIT monoclonal antibody ADI-55812 | YAFTGYYMH | 19 |
| HCDR2 of the heavy chain variable region of the anti-TIGIT monoclonal antibody ADI-55812 | SIIPFSGEANY AQKFQG | 20 |
| HCDR3 of the heavy chain variable region of the anti-TIGIT monoclonal antibody ADI-55812 | ARGPGSLDRLWYYYYGMDV | 21 |
| LCDR1 of the light chain variable region of the anti-TIGIT monoclonal antibody ADI-55812 | RASQSVSSSYLA | 22 |
| LCDR2 of the light chain variable region of the anti-TIGIT monoclonal antibody ADI-55812 | GASSRAT | 23 |
| LCDR3 of the light chain variable region of the anti-TIGIT monoclonal antibody ADI-55812 | QQYGSSPIT | 24 |
| HCDR1 of the heavy chain variable region of the anti-PVRIG monoclonal antibody ADI-56127 | GTESVDAIS | 25 |
| HCDR2 of the heavy chain variable region of the anti-PVRIG monoclonal antibody ADI-56127 | DIIPFFDTDYAQKFQG | 26 |
| HCDR3 of the heavy chain variable region of the anti-PVRIG monoclonal antibody ADI-56127 | AREGGTSWTHFFDL | 27 |
| LCDR1 of the light chain variable region of the anti-PVRIG monoclonal antibody ADI-56127 | RASQSVSSSYLA | 22 |
| LCDR2 of the light chain variable region of the anti-PVRIG monoclonal antibody ADI-56127 | GASSRAT | 23 |
| LCDR3 of the light chain variable region of the anti-PVRIG monoclonal antibody ADI-56127 | QQYGSSPIT | 24 |
| Amino acid sequences of the extracellular domains of human PVRIG | MRTEAQVPALQPPEPGLEGAMGHRTLVLPWV LLTLCVTAGTPEVWVQVRMEATELSSFTIRCG FLGSGSISLVTVSWGGPNGAGGTTLAVLHPER GIRQWAPARQARWETQSSISLILEGSGASSPCA NTTFCCKFASFPEGSWEACGSLPPSSDPGLSAP PTPAPILRAD | 28 |
| Amino acid sequences of the extracellular domains of cynomolgus PVRIG | MRTEAQVLALQSPEPGLEGAMGRRTLALPWV LLTLCVTAGTPEVWVQVQMEATELSSFTVHC GFLGPGSISLVTVSWGGPDGAGGTKLAVLHPE LGTRQWAPARQARWETQSSISLALEDSGASSP FANTTFCCKFASFPEGSWESCGSLPPSSDPGLS APPTPVPILRAD | 29 |
| Amino acid sequences of the extracellular domains of mouse PVRIG | SPEVWVQVQMEATNLSSFSVHCGVLGYSLISL VTVSCEGFVDAGRTKLAVLHPEFGTQQWAPA RQAHWETPNSVSVTLTMGQSKARSSLANTTFC CEFVTFPHGSRVACRDLHRSDPGLSAPTPALN LQAD | 30 |
| Amino acid sequences of the extracellular domains of human TIGIT | MMTGTIETTGNISAEKGGSIILQCHLSSTTAQV TQVNWEQQDQLLAICNADLGWHISPSFKDRV APGPGLGLTLQSLTVNDTGEYFCIYHTYPDGT YTGRIFLEVLESSVAEHGARFQIP | 31 |
| Amino acid sequences of the extracellular domains of cynomolgus TIGIT | MMTGTIETTGNISAKKGGSVILQCHLSSTMAQ VTQVNWEQHDHSLLAIRNAELGWHIYPAFKD | 32 |
| | RVAPGPGLGLTLQSLTMNDTGEYFCTYHTYPD GTYRGRIFLEVLESSVAEHSARFQIP | |
| Amino acid sequences of the extracellular domains of mouse TIGIT | TIDTKRNISAEEGGSVILQCHFSSDTAEVTQVD WKQQDQLLAIYSVDLGWHVASVFSDRVVPGP SLGLTFQSLTMNDTGEYFCTYHTYPGGIYKGR IFLKVQESSDDRNGLAQFQTAPLG | 33 |
| Linker | GGGGSGGGGSG | 44 |
| Flexible linker | GGGGSGGGGSGGGGSGGGGSG | 45 |
| Flexible linker | GGGGSGGGGSGGGGSGGGGS | 46 |
| Sequences of the heavy chain variable region of the anti-TIGIT monoclonal antibody ADI-55796 mutated at position 44 | QLQLQESGPGLVKPAETLSLTCTVSGGSISSYD HYWTWIRQPPGKCLEWIGTVYYSGSTFHNPSL KSRVTIPVDTSKNQFSLKLSSVTAADTAVYYC ARVGPDVSHPPFDYWGQGTLVTVSS | 47 |
| Sequences of the light chain variable region of the anti-TIGIT monoclonal antibody ADI-55796 mutated at position 100 | DIQMTQSPSSLSASVGDRVTITCRASQSISSYLN WYQQKPGKAPKLLIYAASSLQSGVPSRFSGSG SGTDFTLTISSLQPEDFATYYCQQSYSTPITFGC GTKVEIK | 48 |
| Sequences of the heavy chain variable region of the anti-PVRIG monoclonal antibody ADI-56127 mutated at position 44 | QVQLVQSGAEVKKPGSSVKVSCKASGGTESV DAISWVRQAPGQCLEWMGDIIPFFDTDYAQKF QGRVTITADESTSTAYMELSSLRSEDTAVYYC AREGGTSWTHFFDLWGRGTLVTVSS | 49 |
| Sequences of the light chain variable region of the anti-PVRIG monoclonal antibody ADI-56127 mutated at position 100 | EIVLTQSPGTLSLSPGERATLSCRASQSVSSSYL AWYQQKPGQAPRLLIYGASSRATGIPDRFSGS GSGTDFTLTISRLEPEDFAVYYCQQYGSSPITF GCGTKVEIK | 50 |

Nucleic acid encoding peptides of the antibodies were separately constructed into the expression frames of pcDNA3.1 by molecular cloning. TP-007-008 bispecific antibody was produced by transfecting HEK293 cells with pcDNA3.1 vector containing the peptide chain #1 and peptide chain #2 of the anti-TIGIT/PVRIG bispecific antibody TP-007-008, TP-003-009 bispecific antibody was produced by transfecting HEK293 cells with pcDNA3.1 vector containing the peptide chain #1 and peptide chain #2 of the anti-TIGIT/PVRIG bispecific antibody TP-003-009, and TP-001-002-003 bispecific antibody was prepared by transfecting HEK293 cells with pcDNA3.1 vector containing the peptide chain #1, peptide chain #2 and peptide chain #3 of the anti-TIGIT/PVRIG bispecific antibody TP-001-002-003. The supernatant was collected after 5 days of cell culture and the target proteins were isolated and purified using Protein A magnetic beads (purchased from Genscript). The magnetic beads were resuspended (1-4 times the volume of the magnetic beads) with an appropriate volume of binding buffer (PBS+0.1% Tween 20, pH 7.4), then added to the samples to be purified and incubated at room temperature for 1 hour with gentle shaking. The samples were placed on a magnetic rack (purchased from Beaver), the supernatant was discarded, and the magnetic beads were washed 3 times with binding buffer. Elution buffer (0.1 M sodium citrate, pH 3.2) was added in a volume 3-5 times that of the magnetic beads and shaken for 5-10 minutes at room temperature, returned to the magnetic rack, collected, and transferred to a collection tube containing neutralizing buffer (1 M Tris, pH 8.54) and mixed. Three bispecific antibodies TP-007-008, TP-003-009 and TP-001-002-003 were purified, ultrafiltered with buffer replaced by PBS, the molecular weights were confirmed by LC-MS, and then were used for the subsequent in vitro and in vivo detection.

### Example 2: Detection of antibody affinity

Biofilm layer optical interference technology (ForteBio) was used to determine the binding and dissociation rate constant (K_{D}) values of the three bispecific antibodies obtained in Example 1 and their corresponding single-terminal anti-TIGIT monoclonal antibodies ADI-55796, ADI-55812 and anti-PVRIG monoclonal antibody ADI-56127 to human, cynomolgus monkey TIGIT and PVRIG. Fortebio affinity measurements were performed according to known methods (Este, P et al., High throughput solution-based measurement of antibody-antigen affinity and epitope binning. Mabs, 2013.5(2): p270-8). The amino acid sequences of the extracellular region of human, cynomolgus monkey TIGIT and PVRIG were set forth in Table 1.

### The details are as follows:

Measurement of the monovalent affinity of antibodies to human, cynomolgus monkey TIGIT-his and human PVRIG-his proteins: the sensor was equilibrated offline in an analysis buffer for 20 minutes, followed by online detection for 120 s to establish a baseline, and the intact antibodies were loaded onto the AHQ sensor to reach a thickness of 1 nm for affinity detection. The antibody-loaded sensor was incubated in 100 nM target antigen to the plateau phase, and then the sensor was transferred to an analysis buffer and incubated for at least 2 minutes for measuring dissociation rate. The kinetic analysis was performed using a 1:1 binding model.

Measurement of the bivalent affinity of antibodies to cynomolgus monkey PVRIG-Fc proteins: the sensor was equilibrated offline in an analysis buffer for 20 minutes, followed by online detection for 120 s to establish a baseline, and the intact antibodies were loaded onto the AHQ sensor to reach a thickness of 1 nm for affinity detection. The antibody-loaded sensor was continued to be loaded in a high concentration of irrelevant intact antibody for 10 min, saturating the Fc-binding site on the AHQ sensor. The saturated sensor was incubated in 100 nM target antigen to the plateau phase, and then the sensor was transferred to an analysis buffer and incubated for at least 2 minutes for measuring dissociation rate. The kinetic analysis was performed using a 1:1 binding model.

The KD values of the bispecific antibodies and their corresponding single-terminal antibodies bound to human and cynomolgus monkey TIGIT and PVRIG are shown in Table 2 below.

**Table 2: The KD values of the anti-TIGIT-anti-PVRIG bispecific antibodies and the corresponding single-terminal antibodies**

| Antibody | Human TIGIT-his (K_{D}) | Cynomolgus TIGIT-his (K_{D}) | Human PVRIG-his (K_{D}) | Cynomolgus PVRIG-hFc (K_{D}) |
|---|---|---|---|---|
| Anti-TIGIT/PVRIG bispecific antibody TP-007-008 | 1.55E-09 | 6.23E-09 | 1.23E-10 | 2.37E-10 |
| Anti-TIGIT/PVRIG bispecific antibody TP-003-009 | 2.08E-10 | 2.90E-08 | 1.02E-10 | 2.36E-10 |
| Anti-TIGIT/PVRIG bispecific antibody TP-001-002-003 | 1.10E-09 | W.B | 7.47E-11 | 3.10E-10 |
| Anti-TIGIT monoclonal antibody ADI-55796 | 3.22E-10 | 3.12E-09 | N.D | N.D |
| Anti-TIGIT monoclonal antibody ADI-55812 | 1.90E-09 | 3.40E-08 | N.D | N.D |
| Anti-PVRIG monoclonal antibody ADI-56127 | N.D | N.D | 1.44E-10 | 2.67E-10 |

| | | | | |
|---|---|---|---|---|
| Notes: "N.D": Not detected; "W.B": weak binding. | | | | |

The results showed that the anti-TIGIT-anti-PVRIG bispecific antibodies TP-007-008 and TP-003-009 bound to human TIGIT and cynomolgus monkey TIGIT antigens with a monovalent affinity similar to that of their single-terminal antibody, ADI-55796; the anti-TIGIT-anti-PVRIG bispecific antibody TP-001-002-003, which binds to human TIGIT antigen with a monovalent affinity similar to that of its single-terminal antibody, ADI-55812; anti-TIGIT-anti-PVRIG bispecific antibodies TP-007-008, TP-003-009 and TP-001-002-003 bound to human PVRIG antigen with a monovalent affinity similar to that of their single-terminal antibody, ADI-56127; anti-TIGIT-anti-PVRIG bispecific antibodies TP-007-008, TP-003-009 and TP-001-002-003 bound to cynomolgus PVRIG antigen with a bivalent affinity similar to that of their single-terminal antibody, ADI-56127.

### Example 3: Binding and blocking activities of anti-TIGIT-anti-PVRIG bispecific antibodies to CHO cells overexpressing human/cynomolgus/mouse TIGIT

### 3.1 Detection of the binding activities of anti-TIGIT-anti-PVRIG bispecific antibodies to human/cynomolgus/mouse TIGIT overexpressed on CHO cells based on flow cytometry

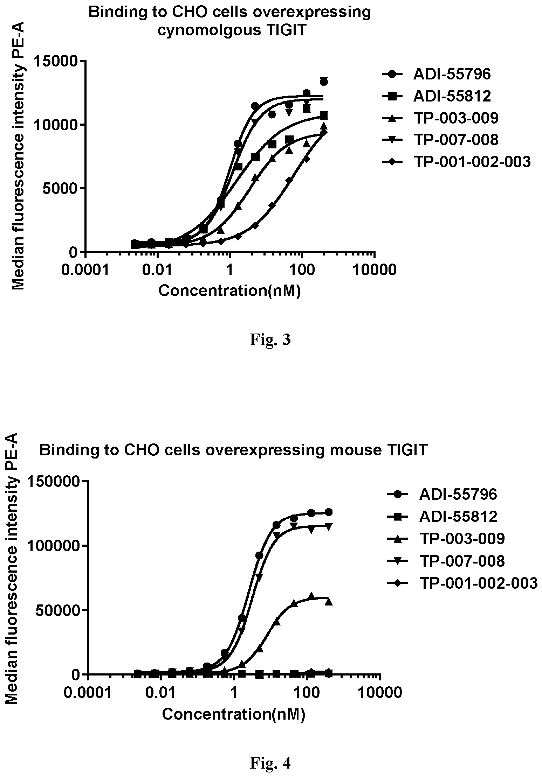

Specifically, CHO cells overexpressing human TIGIT (CHO-huTIGITcells), CHO cells overexpressing cynomolgus TIGIT (CHO-cynoTIGIT cells) and CHO cells overexpressing mouse TIGIT (CHO-muTIGIT cells) were generated by transfection of the pCHO1.0 vector (purchased from Invitrogen) with cDNA encoding human TIGIT, cynomolgus TIGIT and mouse TIGIT cloned into the multiple cloning site (MCS), respectively, followed by pressure screening. The overexpressing cells were expanded and adjusted to an appropriate cell density, and added to a 96-well flow cytometry plate. After centrifugation, the cells were added with a gradiently diluted sample to be tested, and incubated at 4°C for 30 minutes. The cells were washed twice with PBS, and added with a fluorescent secondary antibody correspondingly diluted to an appropriate concentration, then incubated at 4°C for 30 minutes, and washed twice with PBS. Cells were resuspended with PBS, and detected on a CytoFlex flow cytometer, and the corresponding MFI was calculated. Graphpad software was used for graphical analysis to obtain EC₅₀ values. The results were shown in Table 3, Fig. 2 to Fig. 4.

**Table 3. the binding and blocking activities of anti-TIGIT-anti-PVRIG bispecific antibodies to TIGIT/CD155**

| Antibody | EC₅₀ of binding to CHO cells overexpres sing human TIGIT (nM) | EC₅₀ of binding to CHO cells overexpress ing cynomolgus TIGIT (nM) | EC₅₀ of binding to CHO cells overexpres sing mouse TIGIT (nM) | IC₅₀ of blocking the binding of human CD155 to CHO cells overexpressing human TIGIT (nM) | IC₅₀ of blocking the binding of mouse CD155 to CHO cells overexpressing mouse TIGIT (nM) |
|---|---|---|---|---|---|
| Anti-TIGIT/PVRIG bispecific antibody TP-007-008 | 1.681 | 1.206 | 3.124 | 0.482 | 0.981 |
| Anti-TIGIT/PVRIG bispecific antibody TP-003-009 | 3.219 | 3.499 | 8.062 | 0.675 | 3.479 |
| Anti-TIGIT/PVRIG bispecific antibody TP-001-002-003 | 4.253 | 60.47 | N.B | 1.396 | N.B |
| Anti-TIGIT monoclonal antibody ADI-55796 | 1.429 | 1.005 | 2.520 | 0.425 | 0.889 |
| Anti-TIGIT monoclonal antibody ADI-55812 | 0.964 | 1.502 | N.B | 0.296 | N.B |

| | | | | | |
|---|---|---|---|---|---|
| Note: "N.B": No binding. | | | | | |

The results showed that the anti-TIGIT-anti-PVRIG bispecific antibody TP-007-008 of the present invention and its TIGIT-terminal monoclonal antibody ADI-55796, have similar binding activities to CHO cells overexpressing human/cynomolgus/mouse TIGIT.

### 3.2 Detection of the blocking activities of anti-TIGIT-anti-PVRIG bispecific antibodies in the binding of human CD155 to human TIGIT overexpressed on CHO cells and mouse CD155 to mouse TIGIT overexpressed on CHO cells based on flow cytometry

Specifically, the CHO-huTIGIT cells were expanded and adjusted to a cell density of 2×10⁶ cells/mL, added to a 96-well flow plate at 100 µL/well, and centrifuged for later use. The purified antibody was diluted 3-fold with PBS starting at 400 nM for a total of 12 concentration gradients. The diluted sample was added at 60 µL/well to the cells-carrying 96-well flow plate, and incubated at 4°C for 30 minutes. Human CD155 protein with mouse IgG2a Fc tag was then added at 60 µL/well to reach a final concentration of 2 µg/mL, incubated at 4°C for 30 minutes and washed twice with PBS. APC-conjugated goat anti-mouse IgG antibody diluted 100-fold with PBS was added at 100 µL/well, incubated at 4°C for 30 minutes, and washed twice with PBS. Cells were resuspended with PBS at 100 µL/well, and detected on a CytoFlex flow cytometer, and the corresponding MFI was calculated.

The CHO-muTIGIT cells were expanded and adjusted to a cell density of 2×10⁶ cells/mL, added to a 96-well flow plate at 100 µL/well, and centrifuged for later use. The purified antibody was diluted 3-fold with PBS starting at 400 nM for a total of 12 concentration gradients. The diluted sample was added at 60 µL/well to the cells-carrying 96-well flow plate, and incubated at 4°C for 30 minutes. Mouse CD155 protein with mouse IgG2a Fc tag was then added at 60 µL/well to reach a final concentration of 2 µg/mL, incubated at 4°C for 30 minutes and washed twice with PBS. APC-conjugated goat anti-mouse IgG antibody diluted 100-fold with PBS was added at 100 µL/well, incubated at 4°C for 30 minutes, and washed twice with PBS. Cells were resuspended with PBS at 100 µL/well, and detected on a CytoFlex flow cytometer, and the corresponding MFI was calculated. Graphpad software was used for graphing analysis to obtain IC₅₀ values.

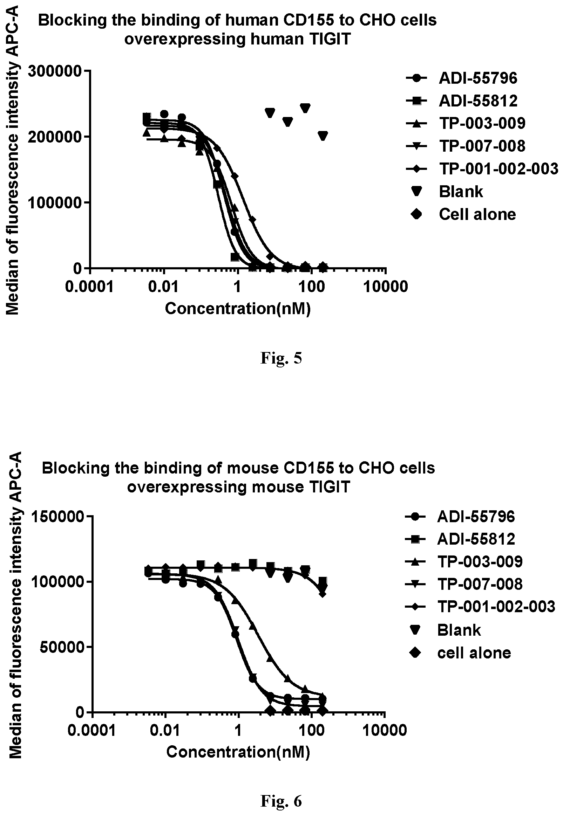

The results were shown in Table 3 and Fig. 5 to Fig. 6. The results showed that the activities of the anti-TIGIT-anti-PVRIG bispecific antibody TP-007-008 of the present invention in blocking the binding of human CD155 to human TIGIT overexpressed on the surface of CHO cells and in blocking the binding of mouse CD155 to mouse TIGIT overexpressed on the surface of CHO cells were comparable to its TIGIT-terminal monoclonal antibody, ADI-55796.

### Example 4: Binding and blocking activities of anti-TIGIT-anti-PVRIG bispecific antibodies to CHO cells overexpressing human/cynomolgus/mouse PVRIG

### 4.1 Detection of the binding activities of anti-TIGIT-anti-PVRIG bispecific antibodies to human/cynomolgus/mouse PVRIG overexpressed on CHO cells based on flow cytometry

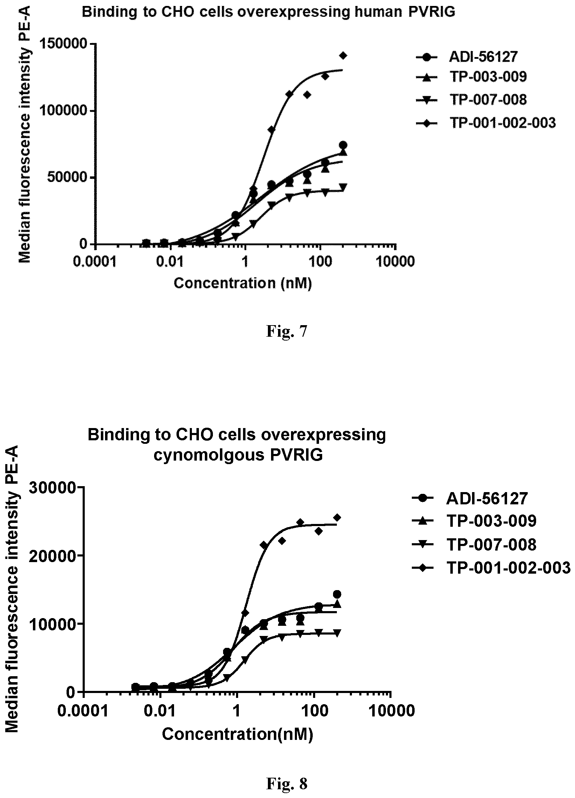

Specifically, CHO cells overexpressing human PVRIG (CHO-huPVRIG cells), CHO cells overexpressing cynomolgus PVRIG (CHO-cynoPVRIG cells) and CHO cells overexpressing mouse PVRIG (CHO-muPVRIG cells) were generated by transfection of the pCHO1.0 vector (purchased from Invitrogen) with cDNA encoding human PVRIG, cynomolgus PVRIG and mouse PVRIG cloned into the multiple cloning site (MCS), respectively, followed by pressure screening. The overexpressing cells were expanded and adjusted to an appropriate cell density, and added to a 96-well flow cytometry plate. After centrifugation, the cells were added with a gradiently diluted sample to be tested, and incubated at 4°C for 30 minutes. The cells were washed twice with PBS, and added with a fluorescent secondary antibody correspondingly diluted to an appropriate concentration, then incubated at 4°C for 30 minutes, and washed twice with PBS. Cells were resuspended with PBS, and detected on a CytoFlex flow cytometer, and the corresponding MFI was calculated. Graphpad software was used for graphical analysis to obtain EC₅₀ values. The results were shown in Table 4, Fig. 7 to Fig. 9.

**Table 4. the binding and blocking activities of anti-TIGIT-anti-PVRIG bispecific antibodies to PVRIG/CD112**

| Antibody | EC₅₀ of binding to CHO cells overexpres sing human PVRIG (nM) | EC₅₀ of binding to CHO cells overexpress ing cynomolgus PVRIG (nM) | EC₅₀ of binding to CHO cells overexpres sing mouse PVRIG (nM) | IC₅₀ of blocking the binding of human PVRIG to CHO cells overexpressing human CD112 (nM) | IC₅₀ of blocking the binding of mouse PVRIG to mouse CD112 protein (nM) |
|---|---|---|---|---|---|
| Anti-TIGIT/PVRIG bispecific antibody TP-007-008 | 2.542 | 1.541 | 17.30 | 1.117 | 0.906 |
| Anti-TIGIT/PVRIG bispecific antibody TP-003-009 | 2.028 | 0.758 | 3.631 | 0.9878 | 0.323 |
| Anti-TIGIT/PVRIG bispecific antibody TP-001-002-003 | 3.113 | 1.717 | 171.9 | 2.004 | 4.487 |
| Anti-PVRIG monoclonal antibody ADI-56127 | 2.542 | 0.844 | 3.346 | 1.061 | 0.261 |

The results showed that the binding activities of the anti-TIGIT-anti-PVRIG bispecific antibody TP-003-009 of the present invention to human/cynomolgus/mouse PVRIG overexpressed on CHO cells is comparable to its PVRIG-terminal monoantibody ADI-56127; the binding activity of the anti-TIGIT-anti-PVRIG bispecific antibody TP-007-008 of the present invention to human PVRIG overexpressed on CHO cells is comparable to its PVRIG-terminal monoantibody ADI-56127.

### 4.2 Detection of the blocking activities of anti-TIGIT-anti-PVRIG bispecific antibodies on the binding of human PVRIG to human CD112 overexpressed on CHO cells based on flow cytometry

Specifically, CHO cells overexpressing human CD112 (CHO-huCD112 cells) were generated by transfection of the pCHO1.0 vector (purchased from Invitrogen) with cDNA encoding human CD112 cloned into the multiple cloning site (MCS), followed by pressure screening. The purified antibody was diluted with PBS and the diluted sample was added to a 96-well flow plate at 60 µL/well. Then, 1 µg/mL of human PVRIG protein with mouse IgG2a Fc tag was added at 60 µL/well, mixed and incubated at 4°C for 30 minutes. The CHO-huCD112 cells were expanded and adjusted to a cell density of 2×10⁶ cells/mL, added to a 96-well flow plate at 100 µL/well, and the supernatant was discarded after centrifugation. The above co-incubated antibody-antigen mixture were added at 100 µL/well to the 96-well flow plate containing CHO-huCD112 cells, incubated at 4°C for 30 min and washed twice with PBS. APC-conjugated goat anti-mouse IgG antibody diluted 100-fold with PBS was added at 100 µL/well, incubated at 4°C for 30 minutes and washed twice with PBS. Cells were resuspended with PBS at 100 µL/well, and detected on a CytoFlex flow cytometer, and the corresponding MFI was calculated. Graphpad software was used for graphical analysis to obtain IC₅₀ values.

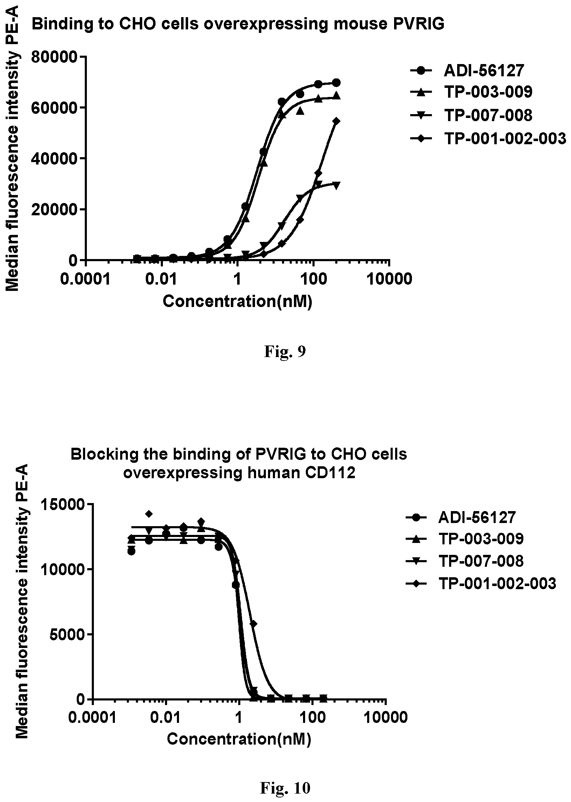

As the results were shown in Table 4 and Fig. 10, the anti-TIGIT-anti-PVRIG bispecific antibodies of the present invention, TP-007-008 and TP-003-009, exhibit similar blocking activity in the binding of human PVRIG to human CD112 overexpressed on CHO cells as their PVRIG-terminal mono-antibody, ADI-56127.

### 4.3 Detection of the blocking activities of anti-TIGIT-anti-PVRIG bispecific antibodies in the binding of mouse PVRIG protein to mouse CD112 protein based on the ELISA method

Specifically, mouse CD112-his protein diluted with 1×ELISA coating buffer to a final concentration of 1 µg/mL was added to the 96-well ELISA plate at 100 µL/well, covered with the film, and coated overnight at 4°C. The coating solution in the ELISA plate was discarded, and the ELISA plate was washed three times with 1×PBST and was blocked with 5% BSA/PBS at 200 µL/well for 2 hours at room temperature. During the blocking period, the purified antibodies to be tested were gradient diluted with 1% BSA/PBS to a final volume of 60 µL/well. Biotin-labeled mouse PVRIG protein with Mouse IgG2a Fc tag was added at 60 µL/well, mixed and incubated for 1 hour at room temperature. The blocking solution in the ELISA plate was discarded, and the above antibody-antigen mixture was added at 100 µL/well and incubated for 2 hours at room temperature. The above antibody-antigen mixture was discarded, the ELISA plate was washed three times with 1×PBST, and SA-HRP diluted with 1% BSA/PBS was added at 100 µL/well and incubated for 1 hour at room temperature. The SA-HRP dilution was discarded, the ELISA plate was washed three times with 1×PBST, ELISA Chromogen Solution was added at 100 µL/well and incubated for 1-3 minutes at room temperature, ELISA stopping solution was added at 50 µL/well and the absorbance values were read at 450 nm. Graphpad software was used to obtain the concentration-absorbance binding curves and the IC₅₀ value.

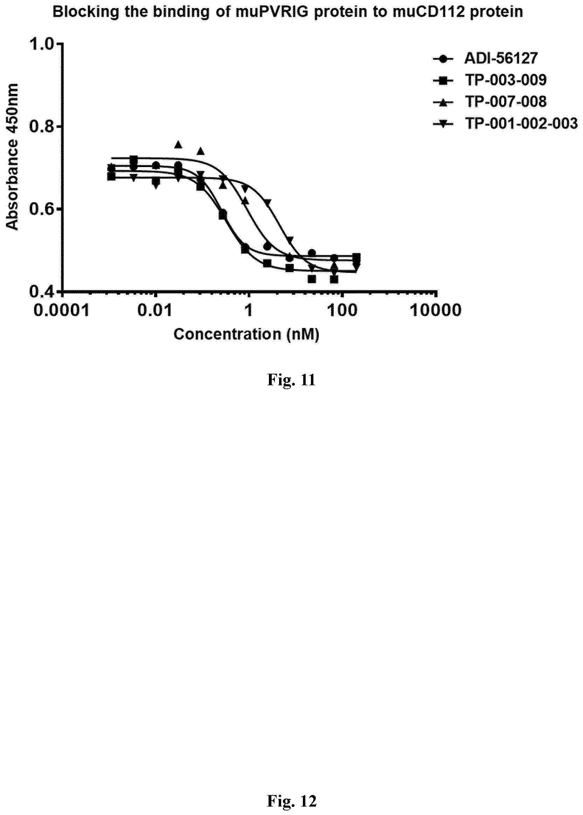

As the results were shown in Table 4 and Fig. 11, the anti-TIGIT-anti-PVRIG bispecific antibody TP-003-009 of the present invention exhibits similar blocking activity in the binding of mouse PVRIG to mouse CD112 protein as that of its PVRIG-terminal monoclonal antibody, ADI-56127.

### Example 5: Co-binding activity of anti-TIGIT-anti-PVRIG bispecific antibody to human TIGIT and human PVRIG

The co-binding activity of the anti-TIGIT-anti-PVRIG bispecific antibody of the present invention to human TIGIT and human PVRIG proteins was detected based on an enzyme-linked immunosorbent assay (ELISA).

Specifically, human PVRIG protein was dissolved according to the instructions, diluted to 1 µg/mL with 1× ELISA coating solution, coated at 100 µL/well in a 96-well ELISA plate, covered with film and placed at 4°C overnight. The coating solution was discarded, washing was performed 3 times with 1×PBST, and 5% BSA/PBS was added at 200 µL/well and blocked at room temperature for 2 hours. The blocking solution was discarded, the antibody to be tested gradiently diluted in 1% BSA/PBS was added at 100 µL/well, and incubated at room temperature for 2 hours. The antibody diluent was discarded, washing was performed 3 times with 1×PBST, biotin-labeled TIGIT protein diluted in 1% BSA/PBS was added at 100 µL/well to a final concentration of 1 µg/mL, and incubated at room temperature for 1 hour. The antigen diluent was discarded, washing was performed 3 times with 1×PBST, SA-HRP diluted in 1% BSA/PBS was added at 100 µL/well, and incubated at room temperature for 1 hour. The SA-HRP diluent was discarded, washing was performed 3 times with 1×PBST, ELISA chromogen solution was added at 100 µL/well and reacted at room temperature for 1 to 3 minutes, then ELISA stop solution was added at 50 µL/well, and the absorbance value at 450 nm was read. Graphpad software was used to plot the concentration-absorbance value binding curve.

The results were shown in Figure 12, the anti-TIGIT-anti-PVRIG bispecific antibodies of the present invention, TP-007-008, TP-003-009 and TP-001-002-003, all could simultaneously bind to human TIGIT and human PVRIG proteins.

### Example 6: Blocking activity of anti-TIGIT-anti-PVRIG bispecific antibody on TIGIT/CD155 and PVRIG/CD112 signaling pathways in luciferase reporter gene system

To further detect the co-blocking activity of anti-TIGIT-anti-PVRIG bispecific antibody on TIGIT/CD155 and PVRIG/CD112 signaling pathways at the cellular level, the following luciferase reporter system was constructed in this example. Briefly, a CHO-K1 cell line (CHO-K1-CD155-CD112-aAPC) overexpressing human CD155, human CD112 and anti-CD3 scFv and a Jurkat cell line (Jurkat-TIGIT-PVRIG-NF-AT-luc) overexpressing human TIGIT, human PVRIG and the companying NF-AT luciferase reporter genes were constructed by transfecting cells with lentiviral. This reporter system was subsequently used to perform relevant experiments.

Specifically, CHO-K1-CD155-CD112-aAPC functional cells were obtained by digestion, adjusted to the desired cell density, added at 100 µL/well to a 96-well white bottom plate, and cultured overnight for attachment. The next day, Jurkat-TIGIT-PVRIG-NF-AT-luc effector cell suspension was prepared, and the samples to be tested were gradiently diluted with reaction medium. The white bottom plate was taken out, the culture supernatant was discarded by pipetting, the above diluted samples were added to the white bottom plate at 40 µL/well, the Jurkat-TIGIT-PVRIG-NF-AT-luc effector cell suspension was added at 40 µL/well at the same time, and the incubation was performed in a 37°C, 5% CO₂ incubator for 6 hours. The Bio-Glo^{™} reagent was returned to room temperature during this period. After incubation, the cells were equilibrated for 5 minutes at room temperature and added with Bio-Glo^{™} reagent at 80 µL/well, and a multifunctional microplate reader was used to read the luminescence signal values.

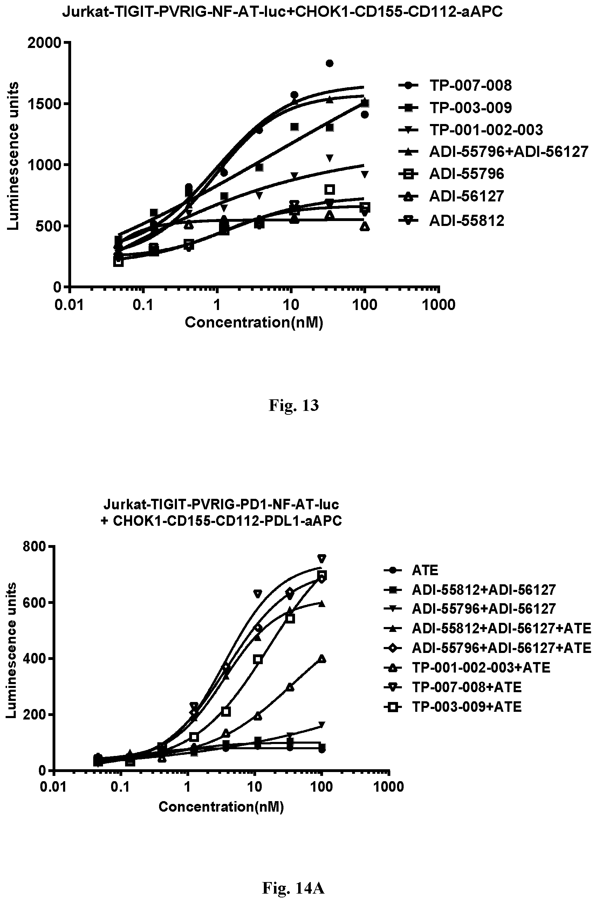

The results were shown in Fig. 13. The results showed that the anti-TIGIT-anti-PVRIG bispecific antibodies of the present invention, TP-007-008, TP-003-009 and TP-001-002-003, were all able to release the TIGIT inhibitory signal mediated by CD155 and the PVRIG inhibitory signal mediated by CD112, and enhanced the expression of the luciferase reporter gene, and the blocking activity of TP-007-008 was comparable to that of the combination of two monoclonal antibodies.

### Example 7: Synergistic blocking effect of anti-TIGIT-anti-PVRIG bispecific antibody with anti-PD-1/L1 antibody in luciferase reporter system

To detect the synergistic blocking activity of anti-TIGIT-anti-PVRIG bispecific antibody with anti-PD-1/L1 antibody at the cellular level, the following luciferase reporter gene system was constructed in this example. Briefly, based on Example 6, lentivirus was used to infect CHO-K1-CD155-CD112-aAPC to overexpress PD-L1 to obtain CHO-K1-CD155-CD112-PD-L1-aAPC functional cells, and lentivirus was used to infect Jurkat-TIGIT-PVRIG-NF-AT-luc to overexpress PD-1 to obtain Jurkat-TIGIT-PVRIG-PD-1-NF-AT-luc effector cells, and this reporter gene system was used in subsequent experiments.

Specifically, CHO-K1-CD155-CD112-PD-L1-aAPC functional cells were obtained by digestion, adjusted to the desired cell density, added at 100 µL/well to a 96-well white bottom plate, and cultured overnight for attachment. The next day, Jurkat-TIGIT-PVRIG-PD-1-NF-AT-luc effector cell suspension was prepared, and the samples to be tested were gradiently diluted with reaction medium. The white bottom plate was taken out, the culture supernatant was discarded by pipetting, the above diluted samples were added to the white bottom plate at 40 µL/well, the Jurkat-TIGIT-PVRIG-PD-1-NF-AT-luc effector cell suspension was added at 40 µL/well at the same time, and the incubation was performed in a 37°C, 5% CO₂ incubator for 6 hours. After incubation, the cells were added with Bio-Glo^{™} reagent at 80 µL/well, and a multifunctional microplate reader was used to read the luminescence signal values.

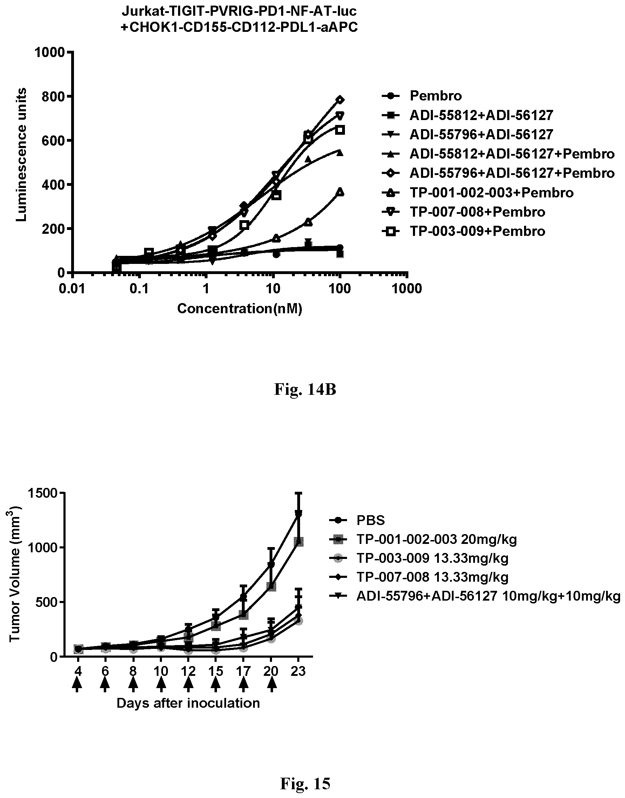

The results were shown in Fig. 14A to 14B. The results showed that the anti-TIGIT-anti-PVRIG bispecific antibodies of the present invention, TP-007-008, TP-003-009 and TP-001-002-003, in combination with the anti-PD-1 antibody Pembrolizumab (Pembro) or with the anti-PD-L1 antibody Atezolizumab (ATE) at a molar ratio of 1:1, could synergistically block the downstream inhibitory signal mediated by PVRIG/CD112, TIGIT/CD155 and PD-1/PD-L1, and further enhance the luciferase signal.

### Example 8: In vivo pharmacodynamic study of anti-TIGIT-anti-PVRIG bispecific antibody in B-NDG mice inoculated with mixed A375 and human PBMCs

In this experiment, an A375 huPBMC model was established in B-NDG mice inoculated with mixed A375 (purchased from Addexbio, Cat. No.: C0020004, a malignant human melanoma cell) and human PBMC cells (Milestone Biotechnologies, A10S033014/PB100C) to determine the anti-tumor effect of the anti-TIGIT-anti-PVRIG bispecific antibody of the present invention. In this regard, a humanized tumor mouse model with a partially recombinant human immune system was generated by inoculating human immune cells (PBMC) into immunodeficient mice.

Specifically, A375 cells were first mixed 1:1 with human PBMCs to form a 0.1 mL cell suspension, and the A375 huPBMC model was established in the right groin of mice by subcutaneous injection. Once the average tumor volume reached about 75 mm³, the mice were divided into groups and intraperitoneally injected with PBS or antibody treatments at different doses but with the same volume of administration. Each group consisted of six mice. The changes in tumor volume and body weight of the mice in each group were monitored with a monitoring frequency of every 2 to 3 days, for 2 to 3 weeks. The dosage and method of administration were shown in Table 5.

**Table 5: Dosage regimen of anti-TIGIT-anti-PVRIG bispecific antibody in A375 huPBMC model**

| Group | Administration dose | Administration frequency |
|---|---|---|
| PBS | N/A | Q2-3d×8 |
| TP-001-002-003 | 20 mg/kg | Q2-3d×8 |
| TP-003-009 | 13.33 mg/kg | Q2-3d×8 |
| TP-007-008 | 13.33 mg/kg | Q2-3d×8 |
| ADI-55796+ADI-56127 | 10 mg/kg+10 mg/kg | Q2-3d×8 |

| | | |
|---|---|---|
| Notes: The molecular weights of TP-001-002-003, TP-003-009, TP-007-008, ADI-55796 and ADI-56127 are approximately 150 KD, 200 KD, 200 KD, 150 KD, and 150 KD, respectively, and the administered dose of each group was an equimolar dose. | | |

The results were shown in Fig. 15. The results showed that the anti-TIGIT-anti-PVRIG bispecific antibodies TP-007-008 and TP-003-009 of the present invention had significant anti-tumor effects, and the tumor suppressive effects were comparable to those of the combination of two single-terminal antibodies.

### Example 9: In vivo pharmacodynamic study of anti-TIGIT-anti-PVRIG bispecific antibody combined with anti-PD-1 monoclonal antibody in B-NDG mice inoculated with mixed A375 and human PBMCs

In this experiment, A375 huPBMC mouse models were established by subcutaneous mixed inoculation (the modeling procedure was the same as in Example 8). Once the average tumor volume reached about 200 mm³, the mice were divided into groups and intraperitoneally injected with PBS or antibody treatments at different doses but with the same volume of administration. Each group consisted of six mice. The changes in tumor volume and body weight of the mice in each group were monitored with a monitoring frequency of every 2 to 3 days, for 2 to 3 weeks. The dosage and method of administration were shown in Table 6.

**Table 6: Experimental protocol for tumor inhibitory activity of anti-TIGIT-anti-PVRIG bispecific antibody**

| Group | Administration dose | Administration frequency |
|---|---|---|
| PBS | N/A | Q2-3d×5 |
| Anti-PD-1 | 5 mg/kg | Q2-3d×5 |
| TP-003-009 | 13.33 mg/kg | Q2-3d×5 |
| TP-007-008 | 13.33 mg/kg | Q2-3d×5 |
| ADI-55796+ADI-56127 | 10 mg/kg+10 mg/kg | Q2-3d×5 |
| Anti-PD-1+ADI-55796 | 5 mg/kg+10 mg/kg | Q2-3d×5 |
| Anti-PD-1+ADI-56127 | 5 mg/kg+10 mg/kg | Q2-3d×5 |
| Anti-PD-1+ADI-55796+ADI- 56127 | 5 mg/kg+10 mg/kg+10 mg/kg | Q2-3d×5 |
| Anti-PD-1+TP-003-009 | 5 mg/kg+13.33 mg/kg | Q2-3d×5 |
| Anti-PD-1+TP-007-008 | 5 mg/kg+13.33 mg/kg | Q2-3d×5 |

In order to further investigate the dose-dependent efficacy of the combination of anti-TIGIT-anti-PVRIG bispecific antibody TP-007-008 and anti-PD-1 monoclonal antibody, the following experimental protocol was designed: the A375 huPBMC model was established by subcutaneous mixed inoculation (the modeling procedure was the same as in Example 8), and the mice were divided into groups when the average tumor volume reached about 300 mm³. Different doses of TP-007-008 combined with a fixed dose of anti-PD-1 antibody were administered by intraperitoneal injection to 6 mice in each group. The changes in tumor volume and body weight of the mice in each group were monitored with a monitoring frequency of every 2 to 3 days, for 2 to 3 weeks. The dosage and method of administration were shown in Table 7.

**Table 7: Experimental protocol for tumor inhibitory activity of anti-TIGIT-anti-PVRIG bispecific antibody**

| Group | Administration dose | Administration frequency |
|---|---|---|
| PBS | N/A | Q2-3d×4 |
| Anti-PD-1+TP-007-008 | 3 mg/kg + 3 mg/kg | Q2-3d×4 |
| Anti-PD-1+TP-007-008 | 3 mg/kg + 12 mg/kg | Q2-3d×4 |
| Anti-PD-1+TP-007-008 | 3 mg/kg + 48 mg/kg | Q2-3d×4 |

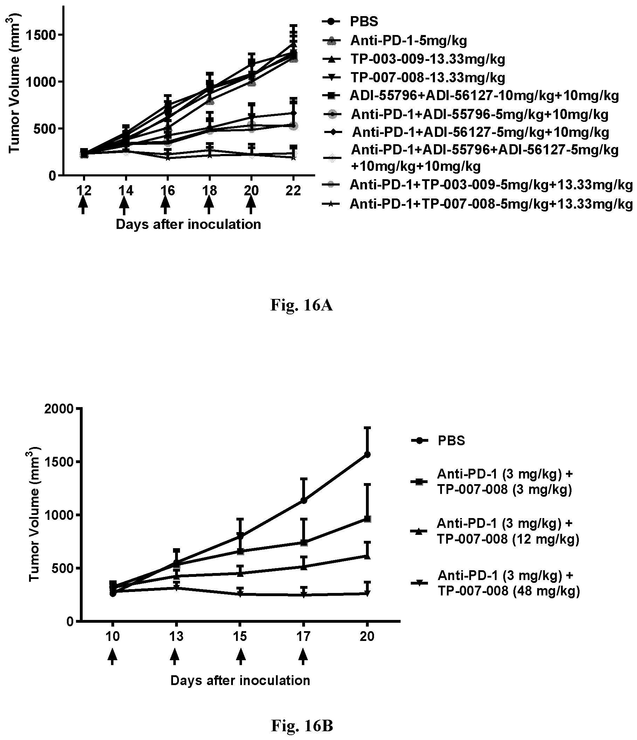

As shown in Fig. 16A, when the initially administered tumor volume reached 200 mm³ or more, no significant tumor inhibitory efficacy was observed in the groups of the anti-TIGIT-anti-PVRIG bispecific antibody TP-007-008 of the present invention, the anti-PD-1 monoclonal antibody, and the combination of the anti-TIGIT and the anti-PVRIG monoclonal antibodies, while the combination of TP-007-008 and the anti-PD-1 monoclonal antibody could significantly inhibit tumor growth and had similar efficacy to the combination of anti-PD-1, anti-TIGIT and anti-PVRIG monoclonal antibodies. As shown in Fig. 16B, the anti-TIGIT-anti-PVRIG bispecific antibody, TP-007-008, in combination with a fixed dose of PD-1 monoclonal antibody (3 mg/kg), had dose-dependent and significant tumor inhibitory activity.

The sequences of anti-PD-1 monoclonal antibody used in this example are shown below:
Amino acid sequence of the VH of anti-PD-1 antibody 
QVQLVQSGAEVKKPGASVKVSCKASGYTFTEYYIYWVRQAPGQGLEWIGGINPSNGGT
NFNEKFKPRVTMTVDTSTSTAYMELSSLRSEDTAVYYCTVRDFRFDKGFKYWGQGTLV
TVSS (SEQ ID NO: 34)
Amino acid sequence of the VL of anti-PD-1 antibody

EIVLTQSPATLSLSPGERATLSCRASKSVSTSGLNYVHWYQRKPGQAPRLLIYLGSYLDS
GVPARFSGSGSGTDFTLTISSLEPEDFAVYYCQQSWELPLTFGGGTKVEIK (SEQ ID NO:
35)

The CDRs for the above anti-PD-1 antibody are listed below:
HCDR1: YTFTEYYIY (SEQ ID NO: 36)
HCDR2: GINPSNGGTNFNEKFKP (SEQ ID NO: 37)
HCDR3: TVRDFRFDKGFKY (SEQ ID NO: 38)
LCDR1: RASKSVSTSGLNYVH (SEQ ID NO: 39)
LCDR2: LGSYLDS (SEQ ID NO: 40)
LCDR3: QQSWELPLT (SEQ ID NO: 41)

The heavy chain constant region of anti-PD-1 monoclonal antibody used in this example was the IgG1 heavy chain constant region modified by L234A, L235A:

ASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKKVEPKSCDKTHTCPPCPAPEAAGG
PSVFLFPPKPKDTLMISRTPEVTCVVVDVSHEDPEVKFNWYVDGVEVHNAKTKPREEQY
NSTYRVVSVLTVLHQDWLNGKEYKCKVSNKALPAPIEKTISKAKGQPREPQVYTLPPSR
DELTKNQVSLTCLVKGFYPSDIAVEWESNGQPENNYKTTPPVLDSDGSFFLYSKLTVDKS
RWQQGNVFSCSVMHEALHNHYTQKSLSLSPG (SEQ ID NO: 42)

The light chain constant region of anti-PD-1 monoclonal antibody used in this example was the human kappa light chain constant region:


 (SEQ ID NO: 43)

### Example 10: In vivo half-life study of the anti-TIGIT-anti-PVRIG bispecific antibody in mice

The in vivo half-life of the anti-TIGIT-anti-PVRIG bispecific antibody of the present invention, TP-007-008, was detected in mice by the tail vein single injection method.

Specifically, Balb/c mice, half male and half female, were used in this experiment and were housed in a 12/12-hour light/dark regulated environment at a temperature of 24°C ± 2°C and a humidity of 40%-70%, with free access to water and food. On the day of the experiment, Balb/c mice received a single tail vein injection of antibody at a dose of 10 mg/kg. Blood collection time points: Blood was collected from the orbits of the mice at 5 minutes, 0.5 hours, 2 hours, 6 hours, 24 hours, 48 hours, 96 hours, 168 hours, 336 hours, and 504 hours after the administration. Whole blood samples were placed at 2°C to 8°C for 30 minutes and centrifuged at 12,000 rpm for 5 minutes to collect serum. The collected serum was centrifuged at 2°C to 8°C, 12,000 rpm for 5 minutes and stored at -80°C. The amount of the bispecific antibody molecules in the serum was detected by ELISA (ELISA assay is the same as example 5).

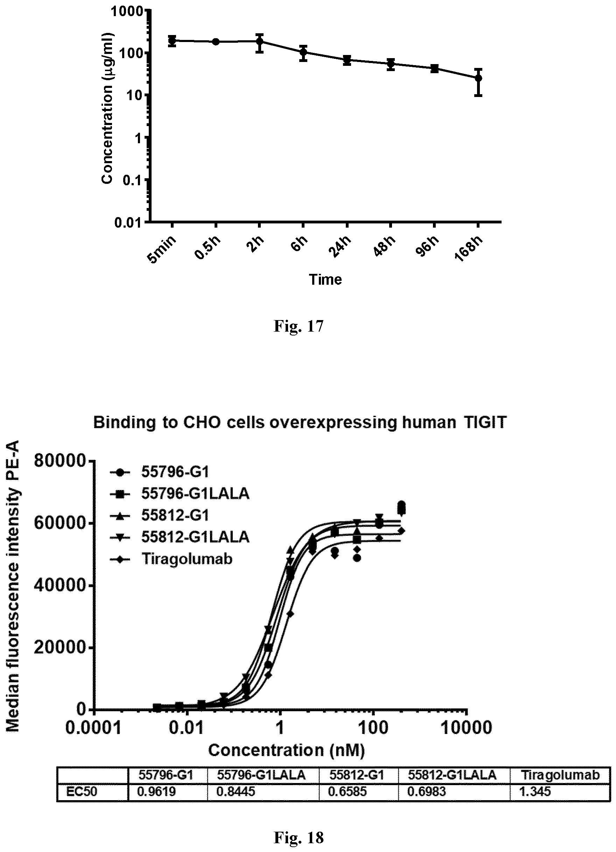

The results were shown in Fig. 17. The results showed that the in vivo half-life of the anti-TIGIT-anti-PVRIG bispecific antibody TP-007-008 of the present invention after a single injection into mice was 103 hours.

### Preparation example 1: Preparation of anti-TIGIT monoclonal antibodies

Nucleic acid encoding the heavy chain variable regions of the anti-TIGIT monoclonal antibody (the amino acid sequence was set forth in SEQ ID NO: 1-2) were constructed into the heavy chain constant region of wild-type human IgG1 and the heavy chain constant region of human IgG1 modified by L234A, L235A, respectively. In addition, nucleic acid encoding the variable regions of the light chain (the amino acid sequence was set forth in SEQ ID NO: 3-4) were constructed into the human immunoglobulin kappa light chain constant region. The anti-TIGIT monoclonal antibodies were designated as 55796-G1, 55796-G1LALA, 55812-G1 and 55812-G1LALA, respectively, and were transiently expressed and purified using the HEK293 expression system. The specific procedures were as follows: the pcDNA3.1 vector containing the heavy and light chains of the antibody was transferred into HEK293 cells by chemical transfection method, cultured at 37°C, 8% CO₂ for 7 days. The cell fluid was collected and centrifuged at 13,000 rpm for 20 minutes. The supernatant was collected, purified with Protein A, and the antibody purity was detected by SEC, while the endotoxin content was controlled. Finally, anti-TIGIT monoclonal antibodies 55796-G1, 55796-G1LALA, 55812-G1 and 55812-G1LALA were obtained.

### Test example 1: Affinity Detection of anti-TIGIT monoclonal antibodies

Biofilm layer optical interference technology (ForteBio) was used to determine the binding and dissociation rate constant (K_{D}) values of the anti-TIGIT monoclonal antibodies obtained in Example 1 to human, cynomolgus monkey and mouse TIGIT. Fortebio affinity measurements were performed according to existing methods (Este, P et al., High throughput solution-based measurement of antibody-antigen affinity and epitope binning. Mabs, 2013.5(2): p270-8). The amino acid sequences of the extracellular regions of human, cynomolgus monkey and mouse TIGIT were shown in Table 1 above.

Measurement of the monovalent affinity of intact antibodies (full-length IgG obtained by Adimab) to human, cynomolgus monkey and mouse TIGIT-his proteins: the sensor was equilibrated offline in an analysis buffer for 20 minutes, followed by online detection for 120 s to establish a baseline, and the intact TIGIT antibodies were loaded onto the AHQ sensor to reach a thickness of 1 nm for affinity detection. The antibody-loaded sensor was incubated in 100 nM TIGIT-his antigen to the plateau phase, and then the sensor was transferred to an analysis buffer and incubated for at least 2 minutes for measuring dissociation rate. The kinetic analysis was performed using a 1:1 binding model.

In the above determination method, the K_{D} values of the antibodies were shown in Table 8:

**Table 8: K_{D} values of anti-TIGIT monoclonal antibodies**

| Antibody | Monovalent affinity of IgG to human TIGIT-his protein | Monovalent affinity of IgG to cynomolgus TIGIT-his protein | Monovalent affinity of IgG to mouse TIGIT-his protein |
|---|---|---|---|
| 55796-G1 | 1.99E-10 | 4.72E-09 | 9.60E-08 |
| 55796-G1LALA | 2.10E-10 | 4.90E-09 | 6.10E-08 |
| 55812-G1 | 3.50E-09 | 2.41E-08 | N.B. |
| 55812-G1LALA | 3.40E-09 | 2.21E-08 | N.B. |
| Tiragolumab | 8.70E-10 | 3.50E-09 | NA |

| | | | |
|---|---|---|---|
| Notes: "N.B.": no binding; "NA": not available. | | | |

It could be seen from the results in Table 8 that: (1) The monovalent affinities of anti-TIGIT monoclonal antibodies to human TIGIT-his protein were higher than that of the control molecule, Tiragolumab; (2) The monovalent affinities of anti-TIGIT monoclonal antibodies to cynomolgus monkey TIGIT-his protein were comparable to that of the control molecule, Tiragolumab; (3) Anti-TIGIT monoclonal antibodies 55796-G1 and 55796- G1LALA showed cross-binding activity with mouse TIGIT.

### Test Example 2: Binding and blocking activities of anti-TIGIT antibodies to CHO cells overexpressing human/cynomolgus/mouse TIGIT

### 2.1 Detection of the binding activities of anti-TIGIT antibodies to human/cynomolgus/mouse TIGIT overexpressed on CHO cells based on flow cytometry.

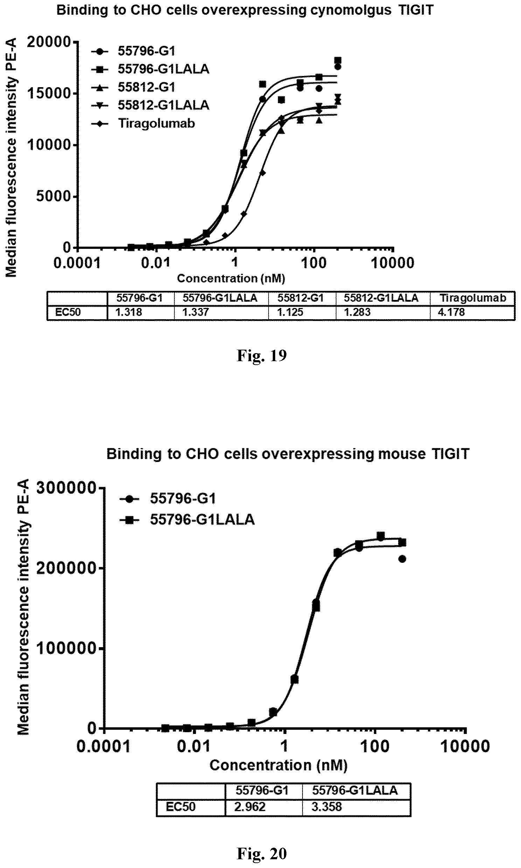

Specifically, CHO cells overexpressing human TIGIT (CHO-huTIGIT cells), CHO cells overexpressing cynomolgus TIGIT (CHO-cynoTIGIT cells) and CHO cells overexpressing mouse TIGIT (CHO-muTIGIT cells) were generated by transfection of the pCHO1.0 vector (purchased from Invitrogen) with cDNA encoding human TIGIT, cynomolgus TIGIT and mouse TIGIT cloned into the multiple cloning site (MCS), respectively, followed by pressure screening. The overexpressing cells were expanded and adjusted to an appropriate cell density, and added to a 96-well flow cytometry plate. After centrifugation, the cells were added with a gradiently diluted sample to be tested, and incubated at 4°C for 30 minutes. The cells were washed twice with PBS, and added with a fluorescent secondary antibody correspondingly diluted to an appropriate concentration, then incubated at 4°C for 30 minutes, and washed twice with PBS. Cells were resuspended with PBS, and detected on a CytoFlex flow cytometer, and the corresponding MFI was calculated. Graphpad software was used for graphical analysis to obtain EC₅₀ values. The results were shown in Table 9, Fig. 18 to Fig. 20.

### 2.2 Detection of the blocking activities of anti-TIGIT antibodies in the binding of human CD155 to human TIGIT overexpressed on CHO cells, and in the binding of mouse CD155 to mouse TIGIT overexpressed on CHO cells based on flow cytometry

Specifically, the CHO-huTIGIT cells were expanded and adjusted to a cell density of 2×10⁶ cells/mL, added to a 96-well flow plate at 100 µL/well, and centrifuged for later use. The purified monoclonal antibodies were diluted 3-fold with PBS starting at 400 nM for a total of 12 concentration gradients. The diluted sample was added at 60 µL/well to the cells-carrying 96-well flow plate, and incubated at 4°C for 30 minutes. Human CD155 protein with mouse IgG2a Fc tag was then added at 60 µL/well to reach a final concentration of 2 µg/mL, incubated at 4°C for 30 minutes and washed twice with PBS. APC-conjugated goat anti-mouse IgG antibody diluted 100-fold with PBS was added at 100 µL/well, incubated at 4°C for 30 minutes, and washed twice with PBS. Cells were resuspended with PBS at 100 µL/well, and detected on a CytoFlex flow cytometer, and the corresponding MFI was calculated.

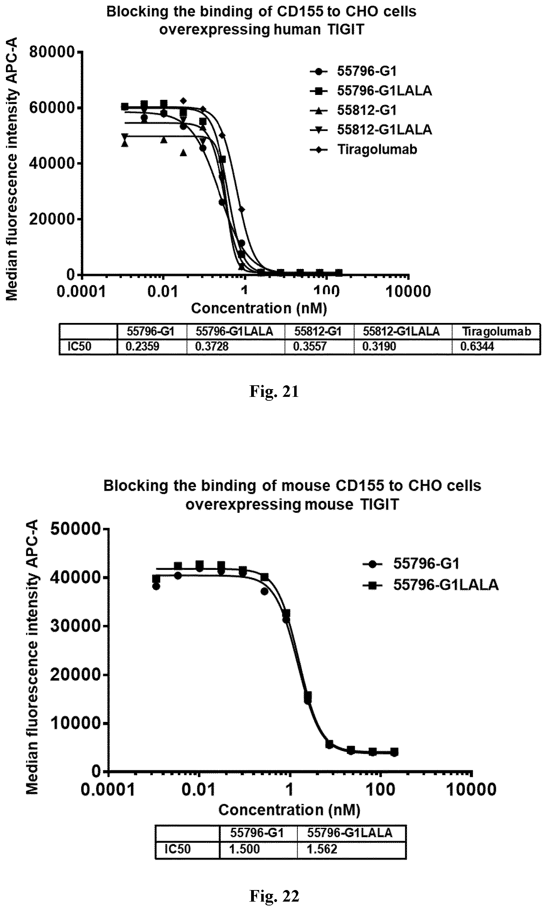

The CHO-muTIGIT cells were expanded and adjusted to a cell density of 2×10⁶ cells/mL, added to a 96-well flow plate at 100 µL/well, and centrifuged for later use. The purified antibodies were diluted 3-fold with PBS starting at 400 nM for a total of 12 concentration gradients. The diluted sample was added at 60 µL/well to the cells-carrying 96-well flow plate, and incubated at 4°C for 30 minutes. Mouse CD155 protein with mouse IgG2a Fc tag was then added at 60 µL/well to reach a final concentration of 2 µg/mL, incubated at 4°C for 30 minutes and washed twice with PBS. APC-conjugated goat anti-mouse IgG antibody diluted 100-fold with PBS was added at 100 µL/well, incubated at 4°C for 30 minutes, and washed twice with PBS. Cells were resuspended with PBS at 100 µL/well, and detected on a CytoFlex flow cytometer, and the corresponding MFI was calculated. Graphpad software was used for graphing analysis to obtain IC₅₀ values. The results were shown in Table 9 and Fig. 21 to Fig. 22.

**Table 9. Summary table of the binding activities and the blocking activities of anti-TIGIT antibodies on overexpressing cells**

| Antibody | EC₅₀ of binding to CHO cells overexpressin g human TIGIT (nM) | EC₅₀ of binding to CHO cells overexpressi ng cynomolgus TIGIT (nM) | EC₅₀ of binding to CHO cells overexpressi ng mouse TIGIT (nM) | IC₅₀ of blocking the binding of human CD155 to CHO cells overexpressi ng human TIGIT (nM) | IC₅₀ of blocking the binding of mouse CD155 to CHO cells overexpressin g mouse TIGIT (nM) |
|---|---|---|---|---|---|
| 55796-G1 | 0.96 | 1.32 | 2.96 | 0.24 | 1.50 |
| 55796-G1LALA | 0.84 | 1.34 | 3.36 | 0.37 | 1.56 |
| 55812-G1 | 0.66 | 1.13 | N.B. | 0.36 | NA |
| 55812-G1LALA | 0.70 | 1.28 | N.B. | 0.32 | NA |
| Tiragolumab | 1.35 | 4.18 | N.B. | 0.63 | NA |

| | | | | | |
|---|---|---|---|---|---|
| Notes: "N.B.": no binding; and "NA": not available. | | | | | |

It could be seen from the results in Table 9, Fig. 18 to Fig. 20 that: (1) The binding activities of the anti-TIGIT antibodies of the present invention to human TIGIT overexpressed on the surface of CHO cells were better than those of the control molecular Tiragolumab; (2) The binding activities of the anti-TIGIT antibodies of the present invention to cynomolgus TIGIT overexpressed on the surface of CHO cells were better than that of the control molecular Tiragolumab; (3) The anti-TIGIT antibodies of the present invention 55796-G1 and 55796-G1LALA showed significant binding to mouse TIGIT protein overexpressed on the surface of CHO cells.

It could be seen from the results in Table 9, Fig. 21 to Fig. 22 that: (1) The activities of the anti-TIGIT antibodies of the present invention in blocking the binding of human CD155 to human TIGIT protein overexpressed on the surface of CHO cells were better than those of the control molecule Tiragolumab; (2) The anti-TIGIT antibodies, 55796-G1 and 55796-G1LALA, which showed binding to the mouse TIGIT protein overexpressed on the surface of CHO cells, could also significantly block the binding of mouse CD155 to mouse TIGIT protein overexpressed on the surface of CHO cells.

### Test example 3: Binding of anti-TIGIT antibody to TIGIT on the surface of primary T cells

The binding activity of the anti-TIGIT antibody of the present invention to TIGIT on the surface of activated T cells was detected based on the flow cytometry detection method.

Specifically, human PBMC were sorted according to the experimental protocol provided by STEMCELL Company (stemcell, Cat. No.: #17951C) to obtain human total T cells. The T cells were adjusted to a concentration of 1.0×10⁶ cells/mL using X-VIVO15 medium (purchased from lonza, Cat. No.: 04-418Q), supplemented with 1 µL of IL-2 stock solution (1,000,000 IU), and simultaneously added with CD3/CD28 Dynabeads (purchased from gibco, Cat. No.: 11132D) at 1:1 ratio (bead-to-cell) and cultured in a 37°C, 5% CO₂ incubator for 48 hours. The activated T cells were adjusted to an appropriate cell density and added to a 96-well flow cytometry plate. After centrifugation, the gradiently diluted sample to be tested was added, and incubated at 4°C for 30 minutes. Washing was carried out twice with PBS, and a fluorescent secondary antibody diluted to an appropriate concentration was added, incubated at 4°C for 30 minutes, and washed twice with PBS. The cells were resuspended with PBS, and detected on a CytoFlex flow cytometer, and the corresponding MFI was calculated.

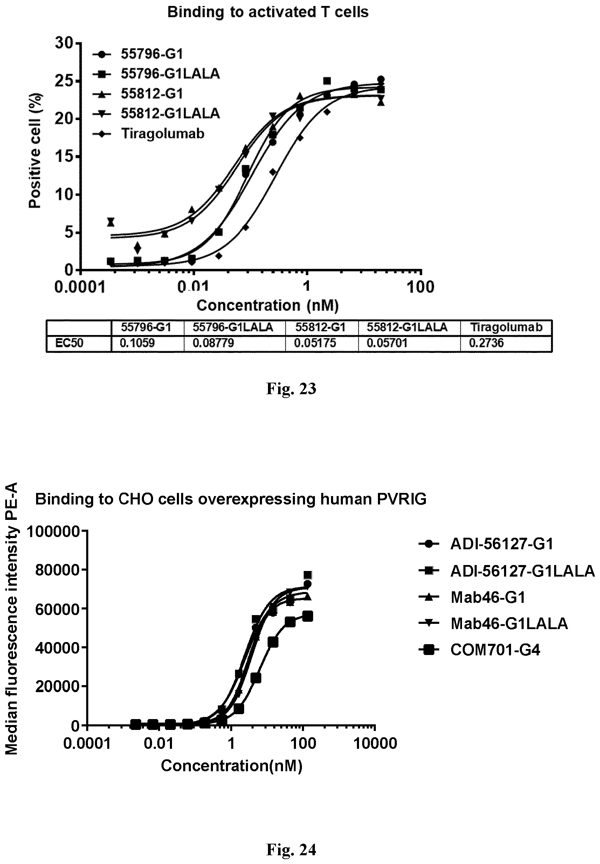

The results were shown in Fig. 23. The results showed that the anti-TIGIT antibodies 55796-G1, 55796-G1LALA, 55812-G1 and 55812-G1LALA of the present invention could bind to TIGIT molecules on the surface of activated T cells, and the binding activities were better than that of the control molecule Tiragolumab.

### Preparation example 2: Preparation of anti-PVRIG monoclonal antibodies

Nucleic acid encoding the heavy chain variable regions of the anti-PVRIG monoclonal antibody (the amino acid sequence was set forth in SEQ ID NO: 5) were constructed into the heavy chain constant region of wild-type human IgG1 and the heavy chain constant region of human IgG1 modified by L234A, L235A, respectively. In addition, nucleic acid encoding the variable regions of the light chain (the amino acid sequence was set forth in SEQ ID NO: 4) were constructed into the human immunoglobulin kappa light chain constant region. The anti-PVRIG monoclonal antibodies were designated as ADI-56127-G1 and ADI-56127-G1LALA, respectively, and were transiently expressed and purified using the HEK293 expression system. The specific procedures were as follows: the pcDNA3.1 vector containing the heavy and light chains of the antibody was transferred into HEK293 cells by chemical transfection method, cultured at 37°C, 8% CO₂ for 7 days. The cell fluid was collected and centrifuged at 13,000 rpm for 20 minutes. The supernatant was collected, purified with Protein A, and the antibody purity was detected by SEC, while the endotoxin content was controlled.

Finally, anti-PVRIG monoclonal antibodies ADI-56127-G1 and ADI-56127-G1LALA were obtained.

### Test example 4: Binding activity and blocking activities of anti-PVRIG antibodies to CHO cells overexpressing human/cynomolgus/mouse PVRIG

### 4.1 Detection of the binding activities of anti-PVRIG antibodies to human/cynomolgus/mouse PVRIG overexpressed on CHO cells based on flow cytometry

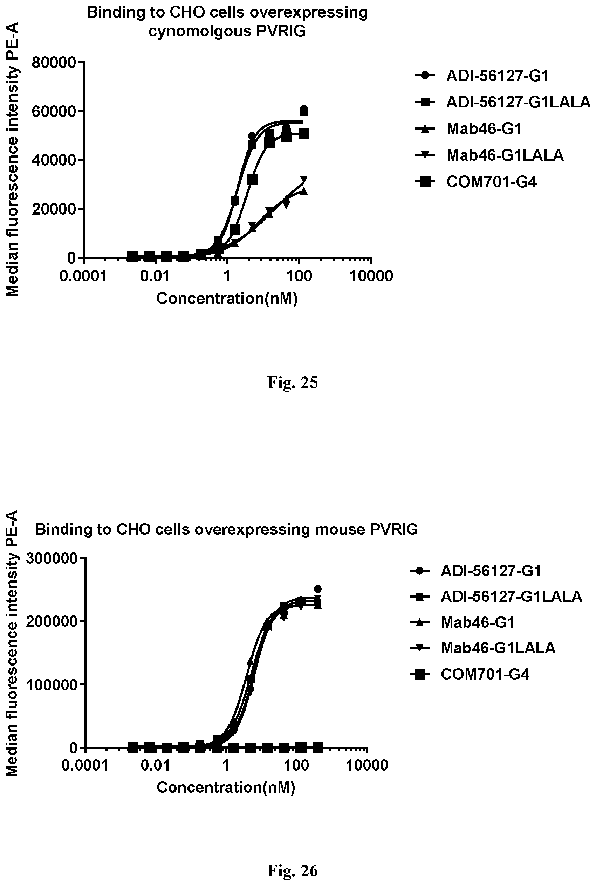

Specifically, CHO cells overexpressing human PVRIG (CHO-huPVRIG cells), CHO cells overexpressing cynomolgus PVRIG (CHO-cynoPVRIG cells) and CHO cells overexpressing mouse PVRIG (CHO-muPVRIG cells) were generated by transfection of the pCHO1.0 vector (purchased from Invitrogen) with cDNA encoding human PVRIG, cynomolgus PVRIG and mouse PVRIG cloned into the multiple cloning site (MCS), respectively, followed by pressure screening. The overexpressing cells were expanded and adjusted to an appropriate cell density, and added to a 96-well flow cytometry plate. After centrifugation, the cells were added with a gradiently diluted sample to be tested, and incubated at 4°C for 30 minutes. The cells were washed twice with PBS, and added with a fluorescent secondary antibody correspondingly diluted to an appropriate concentration, then incubated at 4°C for 30 minutes, and washed twice with PBS. Cells were resuspended with PBS, and detected on a CytoFlex flow cytometer, and the corresponding MFI was calculated. Graphpad software was used for graphical analysis to obtain EC₅₀ values. The results were shown in Table 10, Fig. 24 to Fig. 26.

### 4.2 Detection of the blocking activities of anti-PVRIG antibodies in the binding of human PVRIG to human CD112 overexpressed on CHO cells based on flow cytometry

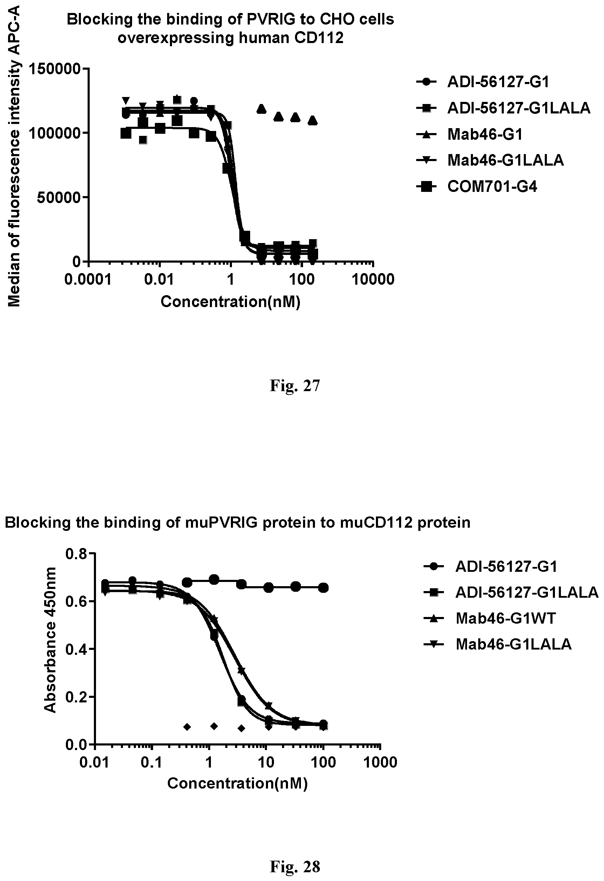

Specifically, CHO cells overexpressing human CD112 (CHO-huCD112 cells) were generated by transfection of the pCHO1.0 vector (purchased from Invitrogen) with cDNA encoding human CD112 cloned into the multiple cloning site (MCS), respectively, follwed by pressure screening. The CHO-huCD112 cells were expanded and adjusted to a cell density of 2 × 10⁶ cells/mL, added to a 96-well flow plate at 100 µL/well, and centrifuged for later use. The purified monoclonal antibody was gradient diluted with PBS and the diluted sample was added to a blank 96-well flow plate at 60 µL/well. Then, 1 µg/mL of human PVRIG protein with mouse IgG2a Fc tag was added at 60 µL/well, and the mixture was mixed and incubated at 4°C for 30 minutes. The above incubated samples were added at 100 µL/well to the 96-well flow plate containing CHO-huCD112 cells, incubated at 4°C for 30 min and washed twice with PBS. APC-conjugated goat anti-mouse IgG antibody diluted 100-fold with PBS was added at 100 µL/well, incubated at 4°C for 30 minutes and washed twice with PBS. Cells were resuspended with PBS at 100 µL/well, and detected on a CytoFlex flow cytometer, and the corresponding MFI was calculated. The results were shown in Table 10 and Fig. 27.

### 4.3 Detection of the blocking activities of anti-PVRIG antibodies in the binding of mouse PVRIG protein to mouse CD112 protein based on the ELISA method

Specifically, mouse CD112-his protein diluted with 1 × coating buffer to a final concentration of 1 µg/mL was added to the ELISA plate at 100 µL/well, covered with the film, and coated overnight at 4°C. The coating solution in the ELISA plate was discarded, and the ELISA plate was washed three times with 1 ×PBST and was blocked with 5% BSA/PBS at 200 µL/well for 2 hours at room temperature. During the blocking period, the samples to be tested were gradient diluted with 1% BSA/PBS to a final volume of 60 µL/well. Biotin-labeled mouse PVRIG huFc protein diluted to 2 µg/mL with 1% BSA/PBS was added to the above sample wells at 60 µL/well, mixed and incubated for 1 hour at room temperature. The blocking solution in the ELISA plate was discarded, and the above sample mixture was added at 100 µL/well and incubated for 2 hours at room temperature. The above co-incubated samples were discarded, the ELISA plate was washed three times with 1×PBST, and SA-HRP diluted with 1% BSA/PBS was added at 100 µL/well and incubated for 1 hour at room temperature. The SA-HRP dilution was discarded, the ELISA plate was washed three times with 1×PBST, ELISA Chromogen Solution was added at 100 µL/well and incubated for 1-3 minutes at room temperature, ELISA stopping solution was added at 50 µL/well and the absorbance values were read at 450 nm. Graphpad software was used to obtain the concentration-absorbance binding curves, the results were shown in Table 10 and Fig. 28.

**Table 10. Summary table of the binding activities and the blocking activities of anti-PVRIG antibodies on overexpressing cells**

| Antibody | EC50 of binding to CHO cells overexpressin g human PVRIG (nM) | EC50 of binding to CHO cells overexpressi ng cynomolgus PVRIG (nM) | EC50 of binding to CHO cells overexpressi ng mouse PVRIG (nM) | IC50 of blocking the binding of human PVRIG to CHO cells overexpressi ng human CD112 (nM) | IC50 of blocking the binding of mouse PVRIG to mouse CD112 protein (nM) |
|---|---|---|---|---|---|
| ADI-56127-G1 | 2.514 | 1.955 | 6.469 | 1.229 | 1.568 |
| ADI-56127-G1LALA | 2.443 | 1.978 | 5.375 | 1.359 | 1.776 |
| Mab46-G1 | 2.999 | 8.18 | 3.877 | 1.153 | 2.834 |
| Mab46-G1LALA | 3.804 | 22.04 | 6.266 | 1.032 | 2.863 |
| COM701-G4 | 6.417 | 3.582 | N.B. | 1.097 | NA |

| | | | | | |
|---|---|---|---|---|---|
| Notes: "N.B.": no binding; and "NA": not available. | | | | | |

The following conclusions could be drawn from Table 10, Fig.24 to Fig. 26:
(1) The binding activities of ADI-56127-G1 and ADI-56127-G1LALA to human PVRIG protein overexpressed on the surface of CHO cells were better than those of the control molecules Mab46-G1, Mab46-G1LALA and COM701-G4;
(2) The binding activities of ADI-56127-G1 and ADI-56127-G1LALA to cynomolgus PVRIG protein overexpressed on the surface of CHO cells were better than those of the control molecules Mab46-G1, Mab46-G1LALA and COM701-G4;
(3) ADI-56127-G1 and ADI-56127-G1LALA showed significant binding to mouse PVRIG protein overexpressed on the surface of CHO cells, and the binding activities is comparable to those of the control molecules Mab46-G1 and Mab46-G1LALA.

The following conclusions could be drawn from Table 10, Fig. 27 to Fig. 28:
(1) The activities of ADI-56127-G1 and ADI-56127-G1LALA in blocking the binding of human PVRIG to human CD112 protein overexpressed on the surface of CHO cells were comparable to those of the of the control molecules Mab46-G1, Mab46-G1LALA and COM701-G4;
(2) ADI-56127-G1 and ADI-56127-G1LALA were able to significantly block the binding of mouse PVRIG to mouse CD112 protein, and the blocking activities were superior to those of the control molecules Mab46-G1 and Mab46-G1LALA.

### Test example 5: Binding of anti-PVRIG antibodies to PVRIG on the surface of primary T cells

The binding activity of the anti-PVRIG antibody of the present invention to PVRIG on the surface of activated T cells was detected based on the flow cytometry detection method.

Specifically, human PBMC were sorted according to the experimental protocol provided by STEMCELL Company (stemcell, Cat. No.: #17951C) to obtain human total T cells. The T cells were adjusted to a concentration of 1.0×10⁶ cells/mL using X-VIVO15 medium (purchased from lonza, Cat. No.: 04-418Q), supplemented with 1µL of IL-2 stock solution (1,000,000 IU), and simultaneously added with CD3/CD28 Dynabeads (purchased from gibco, Cat. No.: 11132D) at a 1:1 ratio (bead-to-cell) and cultured in a 37°C, 5% CO₂ incubator for 48 hours. The activated T cells were adjusted to an appropriate cell density and added to a 96-well flow cytometry plate. After centrifugation, the gradiently diluted sample to be tested was added, and incubated at 4°C for 30 minutes. Washing was carried out twice with PBS, and a fluorescent secondary antibody diluted to an appropriate concentration was added, incubated at 4°C for 30 minutes, and washed twice with PBS. Cells were resuspended with PBS, and detected on a CytoFlex flow cytometer, and the corresponding MFI was calculated.

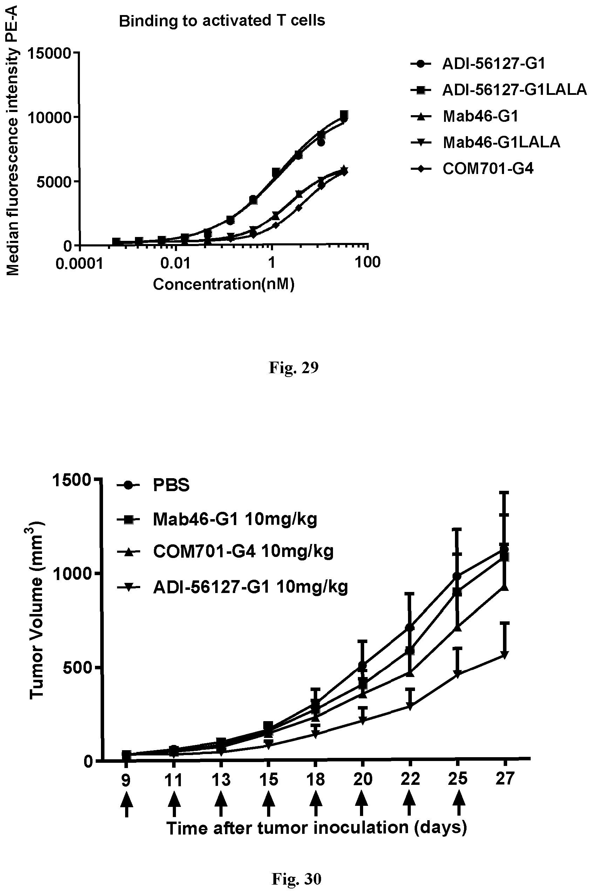

The results were shown in Fig. 29. The results showed that the anti-PVRIG antibodies ADI-56127-G1 and ADI-56127-G1LALA of the present invention could bind to PVRIG molecules on the surface of activated T cells, and the binding activities were better than those of the control molecules Mab46-G1, Mab46-G1LALA and COM701-G4.

### Test example 6: In vivo pharmacodynamic study of anti-PVRIG antibody in NDG mice inoculated with mixed A375 and human PBMCs

In this experiment, an A375 huPBMC model was established in B-NDG mice inoculated with mixed A375 (purchased from Addexbio, Cat. No.: C0020004, a malignant human melanoma cell) and human PBMC cells (Milestone Biotechnologies, A10S033014/PB100C) to determine the anti-tumor effect of the anti-PVRIG antibody of the present invention. In this regard, a humanized tumor mouse model with a partially recombinant human immune system was generated by inoculating human immune cells (PBMC) into immunodeficient mice.

Specifically, A375 cells were first mixed 1:1 with human PBMCs to form a 0.1 mL cell suspension, and the A375 huPBMC model was established in the right groin of mice by subcutaneous injection. Once the average tumor volume reached about 30 to 50 mm³, the mice were divided into groups and intraperitoneally injected with PBS or antibody treatments at different doses but with the same volume of administration. Each group consisted of six mice. The changes in tumor volume and body weight of the mice in each group were monitored with a monitoring frequency of every 2 to 3 days, for 2 to 3 weeks. The dosage and method of administration were shown in Table 11.

**Table 11: Dosage regimen of anti-PVRIG antibody in A375 tumor-PBMC model**

| Group | Administration dose | Administration frequency |
|---|---|---|
| PBS | N/A | Q2-3d×8 |
| ADI-56127-G1 | 10 mg/kg | Q2-3d×8 |
| Mab46-G1 | 10 mg/kg | Q2-3d×8 |
| COM701-G4 | 10 mg/kg | Q2-3d×8 |

The results were shown in Fig. 30. The results showed that the anti-PVRIG antibody ADI-56127-G1 of the present invention can significantly inhibit the growth of A375 tumors in mice, and the anti-tumor activity is superior to that of the control molecules Mab46-G1 and COM701-G4.

Although the specific embodiments of the present invention have been described in detail, those skilled in the art will understand that various modifications and changes can be made to the details based on all teachings that have been published, and these changes are within the protection scope of the present invention. The full scope of the present invention is given by the appended claims and any equivalents thereof.

## Claims

1. A bispecific antibody, which comprises:
A first protein functional region targeting PVRIG, and
A second protein functional region targeting a target (e.g., TIGIT) different from PVRIG;
wherein:
the first protein functional region is an anti-PVRIG immunoglobulin or an antigen-binding fragment thereof;
the heavy chain variable region of the anti-PVRIG immunoglobulin comprises HCDR1 with the amino acid sequence as set forth in SEQ ID NO: 25, HCDR2 with the amino acid sequence as set forth in SEQ ID NO: 26, and HCDR3 with the amino acid sequence as set forth in SEQ ID NO: 27; and
the light chain variable region of the anti-PVRIG immunoglobulin comprises LCDR1 with the amino acid sequence as set forth in SEQ ID NO: 22, LCDR2 with the amino acid sequence as set forth in SEQ ID NO: 23, and LCDR3 with the amino acid sequence as set forth in SEQ ID NO: 24.

2. The bispecific antibody according to claim 1, wherein the second protein functional region is an anti-TIGIT immunoglobulin or an antigen-binding fragment thereof,
wherein:
the heavy chain variable region of the anti-TIGIT immunoglobulin comprises HCDR1 with the amino acid sequence as set forth in SEQ ID NO: 13, HCDR2 with the amino acid sequence as set forth in SEQ ID NO: 14, and HCDR3 with the amino acid sequence as set forth in SEQ ID NO: 15; and the light chain variable region of the anti-TIGIT immunoglobulin comprises LCDR1 with the amino acid sequence as set forth in SEQ ID NO: 16, LCDR2 with the amino acid sequence as set forth in SEQ ID NO: 17, and LCDR3 with the amino acid sequence as set forth in SEQ ID NO: 18;
or,
the heavy chain variable region of the anti-TIGIT immunoglobulin comprises HCDR1 with the amino acid sequence as set forth in SEQ ID NO: 19, HCDR2 with the amino acid sequence as set forth in SEQ ID NO: 20, and HCDR3 with the amino acid sequence as set forth in SEQ ID NO: 21; and the light chain variable region of the anti-TIGIT immunoglobulin comprises LCDR1 with the amino acid sequence as set forth in SEQ ID NO: 22, LCDR2 with the amino acid sequence as set forth in SEQ ID NO: 23, and LCDR3 with the amino acid sequence as set forth in SEQ ID NO: 24.

3. The bispecific antibody according to any one of claims 1 to 2, wherein the antigen-binding fragment thereof is independently a single-chain antibody or an IgG half molecule (IgG-HM).

4. The bispecific antibody according to any one of claims 1 to 3, wherein:
the first protein functional region is an anti-PVRIG immunoglobulin, and the second protein functional region is an anti-TIGIT single-chain antibody; wherein:
the heavy chain variable region of the anti-PVRIG immunoglobulin comprises HCDR1 with the amino acid sequence as set forth in SEQ ID NO: 25, HCDR2 with the amino acid sequence as set forth in SEQ ID NO: 26 and HCDR3 with the amino acid sequence as set forth in SEQ ID NO: 27; and the light chain variable region of the anti-PVRIG immunoglobulin comprises LCDR1 with the amino acid sequence as set forth in SEQ ID NO: 22, LCDR2 with the amino acid sequence as set forth in SEQ ID NO: 23, and LCDR3 with the amino acid sequence as set forth in SEQ ID NO: 24; and
the heavy chain variable region of anti-TIGIT single-chain antibody comprises HCDR1 with the amino acid sequence as set forth in SEQ ID NO: 13, HCDR2 with the amino acid sequence as set forth in SEQ ID NO: 14, and HCDR3 with the amino acid sequence as set forth in SEQ ID NO: 15; and the light chain variable region of the anti-TIGIT immunoglobulin comprises LCDR1 with the amino acid sequence as set forth in SEQ ID NO: 16, LCDR2 with the amino acid sequence as set forth in SEQ ID NO: 17, and LCDR3 with the amino acid sequence as set forth in SEQ ID NO: 18; or
the heavy chain variable region of anti-TIGIT single-chain antibody comprises HCDR1 with the amino acid sequence as set forth in SEQ ID NO: 19, HCDR2 with the amino acid sequence as set forth in SEQ ID NO: 20, and HCDR3 with the amino acid sequence as set forth in SEQ ID NO: 21; and the light chain variable region of the anti-TIGIT immunoglobulin comprises LCDR1 with the amino acid sequence as set forth in SEQ ID NO: 22, LCDR2 with the amino acid sequence as set forth in SEQ ID NO: 23, and LCDR3 with the amino acid sequence as set forth in SEQ ID NO: 24.

5. The bispecific antibody according to any one of claims 1 to 3, wherein:
the first protein functional region is an anti-PVRIG single-chain antibody, and the second protein functional region is an anti-TIGIT immunoglobulin; wherein:
the heavy chain variable region of the anti-PVRIG single-chain antibody comprises HCDR1 with the amino acid sequence as set forth in SEQ ID NO: 25, HCDR2 with the amino acid sequence as set forth in SEQ ID NO: 26, and HCDR3 with the amino acid sequence as set forth in SEQ ID NO: 27; and the light chain variable region of the anti-PVRIG immunoglobulin comprises LCDR1 with the amino acid sequence as set forth in SEQ ID NO: 22, LCDR2 with the amino acid sequence as set forth in SEQ ID NO: 23, and LCDR3 with the amino acid sequence as set forth in SEQ ID NO: 24; and
the heavy chain variable region of the anti-TIGIT immunoglobulin comprises HCDR1 with the amino acid sequence as set forth in SEQ ID NO: 13, HCDR2 with the amino acid sequence as set forth in SEQ ID NO: 14, and HCDR3 with the amino acid sequence as set forth in SEQ ID NO: 15; and the light chain variable region of the anti-TIGIT immunoglobulin comprises LCDR1 with the amino acid sequence as set forth in SEQ ID NO: 16, LCDR2 with the amino acid sequence as set forth in SEQ ID NO: 17, and LCDR3 with the amino acid sequence as set forth in SEQ ID NO: 18; or
the heavy chain variable region of the anti-TIGIT immunoglobulin comprises HCDR1 with the amino acid sequence as set forth in SEQ ID NO: 19, HCDR2 with the amino acid sequence as set forth in SEQ ID NO: 20, and HCDR3 with the amino acid sequence as set forth in SEQ ID NO: 21; and the light chain variable region of the anti-TIGIT immunoglobulin comprises LCDR1 with the amino acid sequence as set forth in SEQ ID NO: 22, LCDR2 with the amino acid sequence as set forth in SEQ ID NO: 23, and LCDR3 with the amino acid sequence as set forth in SEQ ID NO: 24.

6. The bispecific antibody according to any one of claims 1 to 5, wherein:
the heavy chain variable region of the anti-PVRIG immunoglobulin or anti-PVRIG single-chain antibody has an amino acid sequence set forth in SEQ ID NO: 5; and
the light chain variable region of the anti-PVRIG immunoglobulin or anti-PVRIG single-chain antibody has an amino acid sequence set forth in SEQ ID NO: 4;
preferably, the glycine at position 44 of the heavy chain variable region of the anti-PVRIG immunoglobulin or anti-PVRIG single-chain antibody is replaced with a cysteine, and the glycine at position 100 of the light chain variable region of the anti-PVRIG immunoglobulin or anti-PVRIG single-chain antibody is replaced with a cysteine.

7. The bispecific antibody according to any one of claims 1 to 6, wherein:
the heavy chain variable region of the anti-TIGIT immunoglobulin or anti-TIGIT single-chain antibody has an amino acid sequence set forth in SEQ ID NO: 1; and the light chain variable region of the anti-TIGIT immunoglobulin or anti-TIGIT single-chain antibody has an amino acid sequence set forth in SEQ ID NO: 2;
or,
the heavy chain variable region of the anti-TIGIT immunoglobulin or anti-TIGIT single-chain antibody has an amino acid sequence set forth in SEQ ID NO: 3; and the light chain variable region of the anti-TIGIT immunoglobulin or anti-TIGIT single-chain antibody has an amino acid sequence set forth in SEQ ID NO: 4;
preferably, the glycine at position 44 of the heavy chain variable region of the anti-TIGIT immunoglobulin or anti-TIGIT single-chain antibody is replaced with a cysteine, and the glycine at position 100 of the light chain variable region of the anti-TIGIT immunoglobulin or anti-TIGIT single-chain antibody is replaced with a cysteine.

8. The bispecific antibody according to any one of claims 1 to 7, wherein the first protein functional region and the second protein functional region are directly linked or linked via a linker;
preferably, the linker is (GGGGS)m, and m is a positive integer, such as 1, 2, 3, 4, 5, or 6;
preferably, the amino acid sequence of the linker is set forth in SEQ ID NO: 6.

9. The bispecific antibody according to any one of claims 1 to 8, wherein the number of the first protein functional region and the second protein functional region is independently 1, 2, or more than 2.

10. The bispecific antibody according to any one of claims 1 to 9, wherein,
the anti-TIGIT single-chain antibody is respectively linked to the C-terminus of the two heavy chains of the anti-PVRIG immunoglobulin; or
the anti-PVRIG single-chain antibody is respectively linked to the C-terminus of the two heavy chains of the anti-TIGIT immunoglobulin.

11. The bispecific antibody according to any one of claims 1 to 10, wherein,
the constant region of the anti-PVRIG immunoglobulin or anti-TIGIT immunoglobulin is derived from a human antibody;
preferably, the constant region is independently selected from the group consisting of the constant regions of human IgG1, IgG2, IgG3, or IgG4.

12. The bispecific antibody according to any one of claims 1 to 11, wherein,
the heavy chain constant region of the anti-PVRIG immunoglobulin or anti-TIGIT immunoglobulin is human Ig gamma-1 chain C region or human Ig gamma-4 chain C region, and the light chain constant region is human Ig kappa chain C region;
preferably, the heavy chain constant region of the anti-PVRIG immunoglobulin and anti-TIGIT immunoglobulin further comprises a L234A mutation and a L235A mutation according to the EU numbering system.

13. The bispecific antibody according to any one of claims 1 to 12, which is a dimer or tetramer formed with a peptide chain having an amino acid sequence set forth in SEQ ID NO: 7 and a peptide chain having an amino acid sequence set forth in SEQ ID NO: 8, or a dimer or tetramer formed with a peptide chain having an amino acid sequence set forth in SEQ ID NO: 9 and a peptide chain having an amino acid sequence set forth in SEQ ID NO: 10.

14. An isolated nucleic acid molecule, which encodes the bispecific antibody according to any one of claims 1 to 13.

15. A vector, which comprises the isolated nucleic acid molecule according to claim 14.

16. A host cell, which comprises the isolated nucleic acid molecule according to claim 14, or the vector according to claim 15.

17. A conjugate, which comprises a bispecific antibody and a conjugated moiety, wherein the bispecific antibody is the bispecific antibody according to any one of claims 1 to 13, and the conjugated moiety is a detectable label; preferably, the conjugated moiety is a radioactive isotope, a fluorescent substance, a colored substance, or an enzyme.

18. A kit, which comprises the bispecific antibody according to any one of claims 1 to 13, or the conjugate according to claim 17;
preferably, the kit further comprises a second antibody that is capable of specifically binding to the bispecific antibody; optionally, the second antibody further comprises a detectable label, such as a radioactive isotope, a fluorescent substance, a colored substance, or an enzyme.

19. A pharmaceutical composition, which comprises the bispecific antibody according to any one of claims 1 to 13, and one or more pharmaceutically acceptable excipients;
preferably, the pharmaceutical composition further comprises at least one anti-PD-1 antibody;
preferably, the molar ratio of the bispecific antibody to the anti-PD-1 antibody is from (1:5) to (5:1), more preferably 1:1.

20. The pharmaceutical composition according to claim 19, wherein the anti-PD-1 antibody comprises a heavy chain variable region and a light chain variable region, the heavy chain variable region comprises HCDR1 to HCDR3, and the light chain variable region comprises LCDR1 to LCDR3, wherein:
the heavy chain variable region of the anti-PD-1 antibody comprises HCDR1 with the amino acid sequence as set forth in SEQ ID NO: 36, HCDR2 with the amino acid sequence as set forth in SEQ ID NO: 37, and HCDR3 with the amino acid sequence as set forth in SEQ ID NO: 38; and the light chain variable region of the anti-PD-1 antibody comprises LCDR1 with the amino acid sequence as set forth in SEQ ID NO: 39, LCDR2 with the amino acid sequence as set forth in SEQ ID NO: 40, and LCDR3 with the amino acid sequence as set forth in SEQ ID NO: 41;
preferably, the heavy chain variable region of the anti-PD-1 antibody has an amino acid sequence set forth in SEQ ID NO: 34, and the light chain variable region has an amino acid sequence set forth in SEQ ID NO: 35.

21. A combination product, which comprises a first product and a second product in separate packages, wherein:
the first product comprises the bispecific antibody according to any one of claims 1 to 13;
the second product comprises at least one anti-PD-1 antibody;
preferably, the first product and the second product further independently comprise one or more pharmaceutically acceptable excipients;
preferably, the combination product further comprises a package insert;
preferably, the molar ratio of the bispecific antibody to the anti-PD-1 antibody is (1:5) to (5:1), more preferably 1:1.

22. The combination product according to claim 21, wherein the anti-PD-1 antibody comprises a heavy chain variable region and a light chain variable region, the heavy chain variable region comprising HCDR1 to HCDR3, and the light chain variable region comprising LCDR1 to LCDR3, wherein,
the heavy chain variable region of the anti-PD-1 antibody comprises HCDR1 with the amino acid sequence as set forth in SEQ ID NO: 36, HCDR2 with the amino acid sequence as set forth in SEQ ID NO: 37, and HCDR3 with the amino acid sequence as set forth in SEQ ID NO: 38; and the light chain variable region of the anti-PD-1 antibody comprises LCDR1 with the amino acid sequence as set forth in SEQ ID NO: 39, LCDR2 with the amino acid sequence as set forth in SEQ ID NO: 40, and LCDR3 with the amino acid sequence as set forth in SEQ ID NO: 41;
preferably, the heavy chain variable region of the anti-PD-1 antibody has an amino acid sequence set forth in SEQ ID NO: 34, and the light chain variable region has an amino acid sequence set forth in SEQ ID NO: 35.

23. Use of the bispecific antibody according to any one of claims 1 to 13 or the conjugate according to claim 17 in the manufacture of a medicament for the treatment or prevention of a tumor;
preferably, the tumor is one or more selected from the group consisting of colorectal cancer, melanoma, lung cancer, kidney cancer, endometrial cancer, breast cancer, skin cancer, ovarian cancer, stomach cancer, head and neck cancer, liver cancer, brain tumor, urinary tract cancer, bone tumor, bile duct cancer, rectal cancer, pancreatic cancer, cervical cancer, multiple myeloma, Hodgkin's lymphoma, non-Hodgkin's lymphoma, B-cell lymphoma, plasma cell cancer, prostate cancer, and testicular cancer;
preferably, the lung cancer is non-small cell lung cancer or small cell lung cancer.

24. The bispecific antibody according to any one of claims 1 to 13 or the conjugate according to claim 17 for use in the treatment or prevention of a tumor;
preferably, the tumor is one or more selected from the group consisting of colorectal cancer, melanoma, lung cancer, kidney cancer, endometrial cancer, breast cancer, skin cancer, ovarian cancer, stomach cancer, head and neck cancer, liver cancer, brain tumor, urinary tract cancer, bone tumor, bile duct cancer, rectal cancer, pancreatic cancer, cervical cancer, multiple myeloma, Hodgkin's lymphoma, non-Hodgkin's lymphoma, B-cell lymphoma, plasma cell cancer, prostate cancer, and testicular cancer;
preferably, the lung cancer is non-small cell lung cancer or small cell lung cancer.

25. A method for treating or preventing a tumor, comprising a step of administering to a subject in need thereof an effective amount of the bispecific antibody according to any one of claims 1 to 13 or the conjugate according to claim 17;
preferably, the tumor is one or more selected from the group consisting of colorectal cancer, melanoma, lung cancer, kidney cancer, endometrial cancer, breast cancer, skin cancer, ovarian cancer, stomach cancer, head and neck cancer, liver cancer, brain tumor, urinary tract cancer, bone tumor, bile duct cancer, rectal cancer, pancreatic cancer, cervical cancer, multiple myeloma, Hodgkin's lymphoma, non-Hodgkin's lymphoma, B-cell lymphoma, plasma cell cancer, prostate cancer, and testicular cancer;
preferably, the lung cancer is non-small cell lung cancer or small cell lung cancer.
